# EUROPÄISCHE PATENTSCHRIFT

(11) **EP 2 769 696 B1**
(45) Veröffentlichungstag und Bekanntmachung des Hinweises auf die Patenterteilung: **05.12.2018**
(21) Anmeldenummer: 13001398.0
(22) Anmeldetag: 19.03.2013
(51) Int. Cl.: A61B 90/70, A61B 50/33, A61B 50/34, A61B 90/98

(54) **Reinigungsvorrichtung**
Cleaning device
Dispositif de nettoyage

(30) Priorität: 02.02.2013 DE 102013001874
(43) Veröffentlichungstag der Anmeldung: 27.08.2014
(73) Patentinhaber: Simmoteit, Robert, 72414 Rangendingen (DE)
(72) Erfinder: Simmoteit, Robert, 72414 Rangendingen (DE)

(56) Entgegenhaltungen:
- EP-A1- 1 839 683
- WO-A1-03/092524
- WO-A1-2008/136341
- US-A- 5 749 385
- US-A- 5 993 754
- US-A1- 2004 001 776
- US-A1- 2008 267 817
- US-A1- 2010 170 544

## Beschreibung

### Gebiet der Erfindung

Die Erfindung betrifft eine Reinigungsvorrichtung, insbesondere für Reinigungsgeräte, wie z. B. in Reinigungsmaschinen und Ultraschallbädern. Dabei findet die Erfindung überall dort Anwendung, wo Produkte aufbereitet und gelagert werden müssen.

### Stand der Technik

Reinigungsvorrichtungen wie Maschineneinsätze werden in unterschiedlicher Ausgestaltung und Aufbau in Reinigungsmaschinen eingesetzt. Derartige Produkte findet man bei bekannten Herstellern, wie Miele, Bosch, Melag, Belimed, Getinge oder Steelco.

Zum Stand der Technik gehören Reinigungsmaschineneinsätze, so wie in EP2201887 A1 ausgeführt. Hier wird eine Besteckschublade beschrieben sowie Spüleinsätze, welche in einen ausziehbaren Rahmen verschiebbar angeordnet sind. In EP 0186157 A1 wird ebenfalls ein ausziehbarer Besteckkorb mit Aufnahmen für einzelne Besteckteile beschrieben. Andere Besteckschubladen wiederum bestehen aus mehreren Segmenten, die herausnehmbar sind. In DE 43 09 915 A1, der DE 199 35 312 A1 und der US 2005 241682 A1 werden unterschiedliche Besteckschubladen vorgeschlagen, die alle eine Auszugsrichtung und verschiebbare Einsätze besitzen. Auch sind höhenverstellbare Schubladen aus WO 2008 035866 A1 offenbart. Dabei bewegen sich die obigen Einsätze immer horizontal in der Auszugsrichtung.

Im Bereich der medizinischen Instrumentenaufbereitung ist in EP 0186157 A1 ein Tragegestell für die Ablage von Einsatzgestellen zur Aufnahme von unterschiedlichsten Spülgütern vorgesehen und findet Anwendung in Kombination mit einer Maschinensteuerung. Der Einsatz ist hier am Spülwasser-Leitungssystem eines Reinigungsautomaten mit andockbaren Spülwasser-Rohrsystem angeschlossen, um Siebkörbe und dergl. Aufnahmen für die zu reinigenden Spülutensilien aufzunehmen. Derartige Einsätze finden sich auch bei anderen bekannten Herstellen im Bereich der medizinischen Instrumentenaufbereitung. EP 1 839 683 A1 offenbart eine Reinigungsvorrichtung wobei eine Kammer in einem Siebkorb integriert ist.

Ziel aller Vorrichtungen ist, den begrenzt verfügbaren Raum optimal zur Produktreinigung auszunutzen und die Reinigungsmaschinen für die Reinigung möglichst vieler Reinigungsgüter einsetzbar zu gestalten. Reinigungsmaschinen können dabei als Untertisch- oder als Tischmaschinen oder als Ultraschallbäder zum Einsatz kommen.

### Beschreibung

Die Aufgabe der Erfindung betrifft eine Reinigungsvorrichtung, wie z. B. Maschineneinsätze, Siebkörbe und Gitter, insbesondere für den Einsatz in Reinigungs- und Desinfektionsautomaten und Ultraschallbädern, um die Nachteile bekannter Reinigungskörbe, Einsätze verbessern. Diese Nachteile sind beispielsweise: hoher Restwasseranteil in fest installierten Spülmodulen nach der Reinigung, unflexible Ausgestaltung von Siebkörben, Hohlrauminstrumente müssen auf Spezialsiebe umgepackt werden, eine Produktbehandlung muss in unterschiedlichen Reinigungsgeräten erfolgen (z. B. Vorreinigung Ultraschallbad, Hauptreinigung Reinigungsmaschine) oder hohe Druckunterschiede bei der Medienverteilung beeinflussen das Reinigungsergebnis. In Verbindung mit Siebkörben und Gittern oder vergl. Lagerungsvorrichtungen unterstützt die Neuerung die spätere Lagerung der gereinigten Produkte sowie deren Anwendung, z. B. unter sterilen Bedingungen. Weiter ist daran gedacht Maschineneinsätze, einzelne Siebkörbe und/oder Gitter mit Funktionsteilen, Adaptern, Ankopplungssysteme etc. zu kombinieren, so dass auch die Reinigung von Hohlraumprodukten gegenüber bekannten Vorrichtungen verbessert wird. Das hauptsächliche Anwendungsgebiet ist die medizinische Instrumentenaufbereitung. Denkbar ist der Einsatz der Technologie im Labor- und im Haushaltsbereich. Ferner soll die Reinigungsvorrichtung ein durchgängiges Prinzip der Lagerung und Reinigungsmöglichkeiten in einer Maschine und außerhalb dieser ermöglichen sowie unterschiedliche Maschinentechnologie innerhalb einer Aufbereitungskette verbinden.

Die Aufgabe wird erfindungsgemäß durch eine Reinigungsvorrichtung für Reinigungsgeräte nach Anspruch 1 und eine Reinigungsvorrichtung zur Produktlagerung nach Anspruch 8 und Gitter löst. Die abhängigen Ansprüche betreffen vorteilhafte Ausgestaltungen der Erfindung.

Die Erfindung löst die Aufgabe durch eine Reinigungsvorrichtung für Reinigungsgeräte, bei der zumindest ein Maschineneinsatz, ein Einschubwagen, ein Gitter, ein Gitterkorb oder ein Siebkorb oder eine Kombination aus diesen zum Einsatz kommt, dadurch gekennzeichnet, dass die Vorrichtung in oder an einer seitlichen Wand- oder Bodenfläche zumindest eine Austrittsöffnung und/oder einen Ultraschallschwinger besitzt und die Fläche zumindest für den Zeitraum der Überdeckung mit einer Kammerwand oder mit einem Vorbau eine Medien befüllbare Kammer ausbildet, um über die Kammer Medium und/oder Ultraschall für die Reinigung von Produkten zu verteilen. Ein geeigneter Vorbau kann dabei auch permanent vorhanden sein. Dieser Vorbau sollte zumindest lösbar sein, was den Vorteil hat, dass innenliegend auswechselbare Filter vorhanden sein können. Ferner kann die Reinigungsvorrichtung so optimal gereinigt und Restwasser entfernt werden. Die Reinigungsvorrichtung kann aber auch eine Reinigungsmaschinenkammer oder ein Tauchbad sein und diese Vorrichtungen zumindest eine umrahmte oder eine vertiefte Fläche oder eine Nute und/oder eine Fläche mit zumindest einer innenliegenden bzw. hier vorhandenen Austrittsöffnung besitzen.

Die Neuerung sieht dabei eine Reinigungsvorrichtung vor, die durch Anpressen der Vorrichtung zumindest eine vorzugsweise flächige Überdeckung herstellt wird und dabei zumindest eine Kammer ausbildet, um diese mit Medien füllen zu können. Durch Ausbildung einer flächigen Kammer wird die Medienverteilung ebenfalls flächig organisiert und reduziert damit Mediendruckschwankungen bei der Hohlraumreinigung von medizinischen Instrumenten. Die Organisation der Verteilung übernehmen Sprühdüsen, Adapter, Ventile etc. Diese Verbesserung wird vorzugsweise durch eine große flächige Medienkammer erreicht, die durch Anpressen von geeigneten Siebkorb- oder Gitterflächen etc. hergestellt wird. Beispielsweise kann weiter vorgesehen sein, dass eine ganze Seite eines Maschineneinsatzes eine Kammer ausbildet und eine Kammerseite mehrere Austrittsöffnungen vorzugsweise in Ausgestaltung von Adaptern, Ventilen etc. und/oder flächig verteilte Ultraschallschwinger besitzt. An diese können z. B. Siebkörbe mit einer seitlich Wand umrahmten oder vertieften Fläche, Nute und/oder eine Fläche mit mindestens einer Austrittsöffnung angedrückt werden. Eine dichtende Überdeckung (z. B. über eine Dichtung im Randbereich oder eine Dichtfläche) ist bevorzugt, weil hiermit eine Spülvorrichtung ähnlich die eines Spülzylinders und/oder einer Spülleiste hergestellt wird. Ziel ist, ohne zusätzliches Verteilerzubehör, wie Spülrohre oder Spülblöcke oder ähnlichem, auszukommen und vorzugsweise über mehrere Adapter oder Öffnungen in einer Fläche zumindest Medien aus- und weiterzuleiten.

Die Neuerung ist auch für sich alleine zumindest als Maschineneinsatz oder als Gitter, Gitterkorb oder als Siebkorb zur Lagerung und/oder Reinigung von Produkten nutzbar und hat Vorteile gegenüber einem langen Rohrverteiler. Es kann auch vorgesehen sein, dass zumindest eine Wandung einer Gerätekammer oder ein Boden doppelwandig vorhanden ist, um Medium flächig zu verteilen oder auszuleiten. In der Doppelwandung können Heizstäbe oder flächig verteilte Sprühdüsen und/oder Austrittsöffnungen zur Verbesserung der Produktreinigung vorhanden sein.

Durch die vielseitigen Gestaltungsvarianten und deren Nutzung der Neuerung, wird die Wirtschaftlichkeit der Reinigungsprozesse insbesondere dadurch verbessern, dass die Reinigungsvorrichtungen bzw. Reinigungskörbe sich zum Spülmodul wandeln können und eine Hohlraumspülung und/oder eine Ultraschallreinigung ermöglichen. Weiter werden die Anschlussmöglichkeiten von z. B. mobilen oder festeinbaubaren Spülmodulen in und außerhalb einer Reinigungsmaschine verbessert.

Die Neuerung zielt darauf ab, dass eine großflächigere Rahmung und/oder Vertiefung vorhanden ist, als notwendig wäre, um zumindest einen üblichen Medienanschluss zu ermöglichen. Dabei können auch zwei Rahmungen oder zwei Vertiefungen sich gegenseitig überdecken oder die Rahmung kann dabei auch nur eine umlaufende Dichtung oder die Vertiefung kann alternativ auch nur eine Nute sein. Es ist grundsätzlich vorgesehen, dass in der Rahmung und/oder der Vertiefung zumindest eine innenliegende Austrittsöffnung z. B. ein Durchbruch, ein Adapter oder ein Medienanschluss vorhanden ist. Vorzugsweise werden mehrere Adapter in der Fläche angeordnet, um die unterschiedlichsten Hohlraumprodukte wirtschaftlich reinigen zu können. Weiter kann über eine Wandung mit einem Ultraschallschwinger Ultraschall und/oder über eine Öffnung Medium einleitbar sein. Alternativ können hier Ventile oder Steckanschlüsse vorhanden sein, die erst geöffnet werden, wenn hieran etwas angeschlossen wird.

Die benötigte flächige umrahmte und wand- und bodenständige Rahmung oder Vertiefung kann 5 - 20 % und mehr einer zur Verfügung stehenden Wand- oder Bodenfläche einer Reinigungsmaschinenkammer, eines Maschineneinsatzes, eines Gitters, eines Gitterkorbs bzw. eines Siebkorbs sein.

Die Rahmung oder Vertiefung (auch in Ausgestaltung einer Mulde etc.) kann rund, eckig oder eine andere Form aufweisen und eine Wandhöhe oder Wandtiefe von ca. 5 - 100 mm aufweisen. Dabei kann die Rahmung oder Vertiefung eine Dichtung besitzen, selbst eine Dichtung sein oder wie eine Ziehharmonika (gefaltete Rahmung) oder über das Material zusammendrückbar sein. Ziel der Rahmung ist eine Kammer durch Überdeckung ausbilden zu können, dass Medien sicher ein- bzw. ausleitbar sind und darüber hinaus über flächig angeordnete Ultraschallschwinger einen Ultraschall applizierbar wird. Sinnvoll ist, wenn eine Kammer oder Vorkammer entsteht, die dicht genug ist, um Medien weiterleiten oder zeitweise speichern zu können. Für einen derartigen Wandkontakt braucht man keinen Kopplungsadapter, der nach dem Schlüssel-Schloss-Prinzip aufgebaut ist. Damit unterscheidet sich die Neuerung von allen bekannten Reinigungsvorrichtungen, da diese immer mit einer Steckkopplung arbeiten.

Die geeigneten umrahmten seitlichen Flächen und/oder Bodenflächen sollten zumindest nach hinten einen Durchbruch, eine Öffnung, einen Anschluss, ein Ventil, eine Sprühdüse einen Adapter oder einen Aus- bzw. Einleitungskanal oder ähnliches aufweisen. Beispielsweise kann die Rahmung eine umlaufende Dichtung oder eine dichtende Manschette besitzen oder aufnehmen. Alternativ kann die hintere Fläche eine Gitterstruktur sein (innenliegende Öffnungen), wenn nur vorgesehen ist, einen Ultraschall zur Reinigung zu applizieren.

Das Andrücken bzw. die Pressung der Reinigungsvorrichtung an eine Kammerwand oder an einen Kammerboden kann über die Gewichtskraft der Vorrichtung (Maschineneinsatz, Gitterkorb, Siebkorb etc.) oder durch eine mechanische Festlegung in einer Reinigungskammer oder durch Schließen einer Kammertür oder anders erfolgen. Vorzugsweise kann in einem Tauchbad die Gewichtskraft alleine auseichen, damit eine Dichtigkeit mit einer Rahmung zur Weiterleitung von wässrigen Medien entsteht. Zur Unterstützung der Kopplung sind Führungsschienen oder ähnliches in der Reinigungskammer denkbar, um die Reinigungsvorrichtung zu halten und/oder führen zu können.

Die Neuerung zeichnet sich insbesondere dadurch aus, dass die Reinigungsvorrichtungen wie Maschineneinsätze und/oder Gitterkörbe (Siebkörbe etc.) einfach gebaut sind. Ferner ermöglicht diese die Herstellung einer zeitlich begrenzten Spülvorrichtung mit zumindest einer Kammer über die Medien z. B. zur Durchspülung von Hohlrauminstrumenten eingeleitet und gleichmäßig verteilt werden.

Vorteil der Neuerung ist, dass eine kostengünstige und robuste, zum Teil auch wartungsfreie Kopplungsmechanik (einfaches Aufpressen einer Rahmenstruktur) herstellbar ist. Die Reinigungsvorrichtung hat weiter ihre Bedeutung darin, dass eine funktionierende Spülvorrichtung erst dann entsteht, wenn eine Kopplung erfolgt ist. Alternativ ist aber auch denkbar, dass die Rahmung beispielsweise durch Festlegung zweier Siebkörbe oder von Zusatzteilen, wie Platten oder weitere Rahmen, eine Kammer entsteht und diese zumindest von wässrigen Medien durchströmbar ist. Stirnseitig verschraubte Kammern mit einem enthaltenden Ultraschallschwinger sind geeignet einen reinigungswirksamen Ultraschall Siebkorb- und/oder Produktbezogen zu applizieren. Derartige Vorrichtungen können in einem Reinigungsgerät und/oder aber auch außerhalb eingesetzt sein. Dabei können auch Gitterkörbe, die zusammengesetzt aus mehreren gleichen und/oder ungleichartigen Siebkörben zum Einsatz kommen. Vorstellbar sind auch Siebkörbe, die über Scharniere wie ein Werkzeugkasten aufgebaut sind und durch den reduzierten Platzbedarf die OP-Gestaltung und OP-Führung sowie auch die Produktreinigung verbessert.

Um die Neuerung umsetzen zu können, sind die Gitterkörbe (z. B. Siebkörbe) entsprechend zu ändern. Hier ist vorgesehen, dass zumindest über eine Wandung eines Siebkorbs eine Spülfunktion zur Medienverteilung herstellbar ist und/oder über diese Wandung eine Ultraschallbeschallung auch außerhalb eines üblichen Ultraschallbads durchgeführt werden kann. Die hierzu benötigte Rahmung oder Vertiefung (z. B. Mulde) hat für Gitterkörbe den praktischen Nutzen, das Gitterkörbe bzw. Siebkörbe über die Rahmung oder eine Vertiefung an beabstandeten Seitenwänden tragbar werden. Lästige Bügel können entfallen. Weiter bietet eine Rahmung oder eine Vertiefung den weiteren Vorteil, dass zusätzlich ein Flüssigkeitsfilter integrierbar ist. Derartige Filter können Metallgitter oder anders gestaltet sein. Von Vorteil ist, wenn die Filter entnehmbar und wieder reinigbar sind.

Denkbar ist, dass bestehende Reinigungsgeräte zusätzlich zu einer flüssigkeitsbasierten Reinigung insbesondere für Hohlrauminstrumente, eine Ultraschallanwendung in einer Maschinenkammer aufnehmen können. Je nach Ausgestaltung der Reinigungsvorrichtung, insbesondere eines Maschineneinsatzwagens, kann über diesen in unterschiedlichen Etagen einer Reinigungsmaschine Ultraschall appliziert werden. Hierbei wird z. B. ein Siebkorb in einem nach oben hin offenen Behältnis (Kammer) eingebracht. Nach dem sich das Behältnis mit Flüssigkeit gefüllt hat, kann der Ultraschall eingebracht werden. Damit die Flüssigkeit wieder ablaufen kann, besitzt der mit Flüssigkeit füllbare Raum oder die Kammer eine oder mehrere kleine Öffnungen, damit diese sich vorzugsweise langsam entleert. Die Befüllung kann über einen Dreharm und/oder über die Eintrittsöffnung für Medien erfolgen. Somit ist es möglich in Bereichen einer Reinigungsmaschinen (Reinigungs- und Desinfektions-Automaten, RDG), auch in Verbindung mit Ausleitungen, Spüladaptern etc. eine Hohlraumdurchspülung und gleichzeitig eine Ultraschallbehandlung durchzuführen. Hiermit kann der Ultraschall Hohlräume über seine äußere Wirkung reinigen und der innenlumige Schmutz wird durch eine angelegte Durchströmung und chemische Nachreinigung entfernt.

Weiter ist denkbar, dass Gitterkörbe, wie Siebkörbe, auch in einem Waschbecken nutzbar sind. Das wird möglich, wenn ein Siebkorb bzw. Siebkorbteil mit eine Ultraschall applizierende Einheit gekoppelt ist. Ist ausreichend Flüssigkeit im Becken vorhanden, so kann der Ultraschal seine reinigende Wirkung ausüben.

Zur Funktionsverbesserung kann die Vorrichtung einen Transponder oder einen Ultraschallabstandssensor etc. enthalten, damit die Kopplung dokumentierbar ist und/oder eine unkorrekte Kopplung angezeigt wird. Über diese Art der Kennung, kann auch die Maschine gesteuert werden und/oder die benötigte Pumpleistung abgerufen oder die Ultraschalleinheit angesteuert werden.

Damit unterstützt die Vorrichtung die Herstellung geradliniger Aufbereitungsprozesse und die Verbindung unterschiedlicher Behandlungsmethoden, ohne die Maschinentechnologie zu wechseln. Weiter können insbesondere medizinische Holrauminstrumente oder schwierig zu reinigende Produkte über eine Vorrichtung aufbereitet werden und ein optimales Reinigungsergebnis ermöglichen. Die Neuerung kann ein wichtiger Bestandteil einer Prozesskette sein, bei der die Hohlraumproduktreinigung, die Lagerung vor oder nach einer Produktsterilisierung gelöst ist. Kosten- und wirtschaftliche Vorteile ergeben sich weiter dadurch, dass keine zusätzlichen teuren und platzreduzierende Spülmodule eingesetzt werden müssen, da die Neuerung selbst Vorrichtungsteile, wie Kopplungsflächen etc. in Verbindung mit einer Reinigungsvorrichtung, wenn auch nur zweitweise mit änderbaren Reinigungsapplikationen, herstellt.

Die Erfindung ermöglicht weiter eine Reinigungsvorrichtung zur Produktlagerung, insbesondere für Reinigungsmaschinen und Ultraschallbäder, die ein horizontal ausziehbaren Maschinenwagen mit Rollen, ein Korbgitter oder ähnliches mit Rollen oder Schienen aufweisen oder für einen Korb oder Rahmen, der einhängbar ist, dadurch gekennzeichnet ist, dass mehrere Gitter oder Funktionskörbe über einer unteren Lagerungsebene eines Maschineneinsatzes eine hiervon beabstandete und teilbare Lagerungsebene herstellen, um hierauf Waschgutträger und/oder Produkthalterungen über Durchbrüche oder Überdeckungen einer unteren Lagerungsebene festzulegen oder zu positionieren, um zumindest durch Öffnen einer Lagerungsteilfläche hierunter liegende Waschgutträger frei werden, ohne dass die oben gelagerten Waschgutträger und Produkte zuerst entnommen werden müssen.

Die Neuerung ermöglicht hiermit nach dem Baukastenprinzip eine produktbezogene Reinigung und eine ganzheitliche Verknüpfung der Prozessschritte, wie Lagerung, Transport, Desinfektion, Sterilisierung und Anwendung z. B. im OP, ohne einen spezifischen Maschinenwagen für diese Produkte einsetzen zu müssen.

Geeignete Waschgutträger sind beispielsweise Siebkörbe oder Siebkorbbehälter mit oder ohne Füße. Alternativ können die Waschträger auch Drahtgestelle, einfache Rahmen oder Lochkästen sein. In der Medizintechnik werden Silikonhalterungen in den unterschiedlichsten Ausgestaltungen, Formen oder Größen eingesetzt, um Produkt zu lagern oder zu fixieren.

Als Überdeckungen können selbst Gitter, Bügel oder o. ä. von oben aufliegende Komponenten sein, damit hier beispielsweise Siebkörbe oder Produkte auf einer oberen teilbaren Lagerungsebene bzw. Lagerungsteilfläche festlegebar oder zumindest durch Überdeckung so auf einer unteren Lagerungsebene positionierbar sind. Ferner wird dadurch das Hochklappen der Gitter von mehr als 10 Grad ermöglicht, ohne dass die hierauf gelagerten Produkte oder Waschträger herunter fallen oder herunter rutschen können. Es ist dabei vorgesehen, dass zumindest die Bügel am Gitter über Gelenke oder bewegliche Befestigungen drehbar angeordnet sind.

Als Gitter werden bevorzugt Blech- oder Drahtgestelle eingesetzt. Diese können stapelbar und/oder auch mit Rollen oder unteren Puffern ausgestaltet sein. Denkbar ist auch, dass die Gitter selbst korbähnlich (Funktionssiebkörbe) ausgestaltet sind, d. h. mit einem umlaufenden grobmaschigen Gitter, damit Reinigungsmedium diese gut durchdringen kann. An diesem Gitter werden beispielsweise drehbar Bügel oder, Überdeckungen oder elastische Bügel, z. B. aus Silikon, angebracht. Letztere verbessern die Lagerung und die Entnahme gestapleter Siebkörbe in einem Sterilbehälter.

Als Gitter werden Gitter oder als Funktionssiebkörbe werden Körbe bezeichnet, die in erster Linie eine Lagerungsebene herstellen und die Aufbereitung unterstützen. Ferner bieten diese zusätzlichen Fixierungsmöglichkeiten für Halterungsteile (Silikonbänder etc.) oder für Spülmodule oder Fußteile und sind auch außerhalb einer Reinigungsmaschine und/oder in Ultraschallbädern mit oder ohne Durchspülung von Hohlrauminstrumenten einsetzbar. Geeignete Fußteile können wiederum durch Silikonscheiben gebildet sein, damit derartige Gitter rollbar oder in einer Rahmenkonstruktion als Stopper eingebracht sind. Der Begriff Funktion gibt an, dass das Gitter wie ein Baustein nutzbar ist, um die technischen Gestaltungsmöglichkeiten in einer Reinigungsvorrichtung oder -maschine und/oder auch außerhalb derartiger Maschinen zu ändern. Alle Gitter sind tragbar und können auch außerhalb einer Maschine zur Lagerung von Produkten in Verbindung mit Produkthalterungsteilen eingesetzt werden. Es ist vorgesehen, dass derartige Gitter als einfache Rahmen mit Querverstrebungen ausgebildet sind. Querverstrebungen wiederum können dabei ebenfalls wieder Gitter sein.

Wie bereits beschrieben, kann die Neuerung in Ausgestaltung eines Gitters, zumindest an einer Seite eine Rahmung oder eine Vertiefung aufweisen, um diese an eine Reinigungskammerwandung zu pressen, um damit eine Spülvorrichtung herzustellen. Derartige Gitter können somit ohne zusätzliche Spülmodule auskommen oder zumindest deren Einsatz verbessern helfen. Alternativ kann vorgesehen sein, dass ein mobiler Medienverteiler direkt oder über einen Schlauch an einer Austrittsöffnung am Gitter mit Reinigungsmedium für Hohlraumprodukte angebracht wird. Dabei ist angedacht, dass die unter dem teilbaren Gitter liegenden Produkte, Siebkörbe, Lagerungsvorrichtungen o. ä. so entnehmbar sind, dass die oben aufliegenden Waschträger und/oder Medienverteile zumindest nicht abgenommen werden müssen. Zur Aufnahme ist jedoch vorgesehen, die angekoppelten Medienverteiler oder die anpressbare Rahmung zu entkoppeln.

Weiter kann hier vorgesehen sein, dass am Gitter oder am Funktionssiebkorb eine elektronische oder mechanische Kennung vorgesehen ist, die mit der Reinigungsmaschine oder mit einem Ultraschallgerät kommuniziert. Durch diese Kommunikation erkennt beispielsweise die Maschine, welches Gitter vorhanden ist und kann damit weiter die Reinigungsleistung durch eine Änderung der Pumpleistung und/oder der Beschallung oder der Chemiedosierung beeinflussen. Elektronisch verwertbare Kommunikation ist z. B. dort sinnvoll, wo es gilt, einen Reinigungsnachweis für Hohlraumprodukte oder andere Waschgüter zu führen oder eine gesetzliche durchgängige Dokumentation gefordert wird. Alternativ kann diese Information der Anwender über ein Barcode-System der Maschine mitteilen.

In einer anderen Weiterbildung können unterschiedlich gebaute Gittern in einem Maschineneinsatz änderbare und insbesondere durch Einbringen von Medienverteiler (Spülrohre, Spülzylinder, Dreharme o. ä. Vorrichtungen) eine Beeinflussung des Reinigungsergebnisses oder der Reinigungstechnik vornehmen. Dabei kann eine einfache Reinigungsmaschine mit einem einfach gebauten Reinigungskorb vielseitiger eingesetzt werden als ohne diese Ausbauvarianten. Hier kann vorgesehen sein, dass auf und/oder unter den Gittern, Spülmodule (Spülleisten, Spülzylinder, Dreharme, Sprühdüsen o- ä.) zur Medienverteilung angebracht sind. Diese Ausgestaltung ermöglicht beispielsweise die nachträgliche Ausrüstung bestehender Maschinenwagen, aber auch die von geeigneten Ultraschallbädern, um mit diesen Spülmodulen Anschlussmöglichkeiten (Adapter etc.) bereitzustellen, damit Hohlraumprodukte gereinigt werden können. Die Gitter ermöglichen somit, dass in einer Reinigungsmaschine oder einem Ultraschallbad das Reinigungsmedium in unmittelbarer Produktnähe eingebracht wird oder Hohlraumprodukte gereinigt werden können.

Als technische Weiterbildung sind beispielsweise Maschinenwagen oder Ultraschall-Badebenen vorgesehen, die teilbare Lagerungsebenen besitzen und bei dem auf oder unter einem Gitter ein Dreharm oder ein Medienverteilerrohr liegt. Diese überdecken zur Erhöhung der Ladekapazität Waschträger (Siebkörbe, Wasch-Trays oder Wasch-Gestelle o. ä.), die auf einer unteren Lagerungsebene liegen. Dadurch werden die Reinigungsmöglichkeiten nachträglich in einem Maschinenwagen erweitert und die Lagekapazität derartiger Maschineneinsätze verdoppelt. Ein weiterer Vorteil der teilbaren Lagerungsebenen ist, dass die Gitter oder Funktionskörbe z. B. auf dem Rahmen verschiebbar sein können, um ein Hindurchgreifen in die untere Ebene zu erleichtern.

Die Neuerung wird dort eingesetzt, wo die Wirtschaftlichkeit und die Flexibilität von bereits vorhandenen Reinigungsmaschinen und Ultraschallbädern erhöht werden soll. Die Ausgestaltung einer Reinigungsebene, insbesondere, wenn diese durch mehrere Gitter hergestellt ist, kann durchgängige aber auch eine geteilte Ebene ermöglichen. Letzteres ist wichtig, da unterschiedlich hohe Siebkörbe auf den Reinigungsebenen aufliegen oder unter dieser Ebene abgelegt sein können. Alternativ kann auch vorgesehen sein, dass ein Gitter entnommen wird, um eine freibleibende Gitterebene auszubilden. Dies ist dann notwendig, wenn lange Hohlraumprodukte (länger als eine Ebene hoch sind), senkrecht stehend in Spüladapter eingebracht sind und diese von unten mit Reinigungsmedium versorgt werden.

Damit werden die Gitter in unterschiedlichen Maschinen, wie einem Ultraschallbad und einem RDG, einsetzbar und ermöglichen zumindest mit einem Spülmodul (Spülleiste, Spülzylinder etc.), dass Hohlraumprodukte in beiden Maschinen durchspült werden. Auch dann, wenn diese Maschinen keine Spülmodule besitzen, sondern nur geeignete Anschlussvorrichtungen für diese aufweisen. Dabei können die Spülgüter auch in Waschgutträgern (Siebkörbe) liegen oder eingebracht sein und diese letzten Endes zum Sterilisieren eingesetzt werden. Dieses Vorgehen ist bei besonders verschmutzen medizinischen Instrumenten oder Produkten sinnvoll, wenn ein Reinigungsverfahren nicht ausreicht, um das gewünschte Reinigungsergebnis zu erzielen.

Weiterer Vorteil der Neuerung ist, dass genormte und bekannte Reinigungskörbe, beispielsweise aus dem Haushaltsspülbereich oder der Medizintechnik, nutzbar sind. Hier sind signifikante Kosteneinsparungen möglich, da keine neuen Maschinenwagen hergestellt werden müssen. Durch die Prozessverbindung Reinigung und Lagerung ergeben sich wirtschaftlich erhebliche Vorteile sowie Platzeinsparungen.

Die Neuerung löst die Aufgabe hier über ein Gitter, insbesondere für Reinigungsmaschinen und Ultraschallbäder, die auf einem zumindest horizontal ausziehbarem Korbgitter (Maschinenwagen) oder einem eingehängten Rahmen oder ähnlichem abgelegt und dadurch gekennzeichnet sind, dass diese Gitter Durchdringungen besitzen, um hiermit Waschgutträger zumindest über die Durchbrechungen am Gitter festzusetzen oder über die Durchbrechungen Produkthalterungen fixiert sind oder Gitter durch Überdeckung zumindest eine Medienweiterleitung zur Reinigung von Hohlraumprodukten herstellen. Die Festsetzung ist sinnvoll, damit durch Hochklappen oder Entnahme der Gitter die hier aufliegenden Waschgutträger oder die eingelagerten Produkte bewegt und in einer Schrankschublage oder in einem Sterilcontainer eingelagert oder von einer Weichverpackung umhüllt werden können.

Neu ist, dass die Gitter nicht nur in Verbindung mit Reinigungswagen zur Herstellung einer Ebene verwendet werden, sondern eine durchgängige Aufbereitung (reinigen, transportieren, sterilisieren, eventuell auch eine Nutzung im OP) von zumindest medizinischen Produkten ermöglichen. Auf derartigen Gittern können entweder Siebkörbe o. ä. abgelegt, transportiert oder sterilisiert werden. Oder das Gitter selber besitzt Produkthalterungen und dient als Waschgutträger, der transportiert, lager- und sterilisierbar ist. Zur besseren Stapelbarkeit können die Gitter geeignete Füße oder Ausformungen aufweisen.

In einer Weiterbildung der Neuerung besitzen die Gitter geeignete Lagerungsdurchbrüche oder -erhebungen, um hier Silikonhalterungen oder Füße zur besseren Lagerung der Gitter zu fixieren. Dadurch, dass die Gitter aufnehmbar oder hochklappbar sind, können hiermit Produkte beispielsweise sicher transportiert oder abgelegt werden und somit in Schubladen oder Sterilcontainern rutschfest gelagert werden. Die Rutschfestigkeit wird beispielsweise über die Silikonfüße hergestellt. Rutschfestigkeit ist dort von Vorteil, wenn teure und empfindliche medizinische Instrumente gereinigt, transportiert und gelagert werden müssen. Darüber hinaus werden somit die Sterilcontainerböden nicht verkratzt. Alternativ können die Funktionsgestelle auch in Weichverpackungen verpackt werden, um diese anschließend zu desinfizieren und/oder zu sterilisieren.

Eine übliche Ausgestaltung des Gitters ist 20 - 60 cm lang und 10 - 30 cm breit oder kann DIN-Maße aufweisen. Damit genügend Reinigungsmedium oder die Schallwellen hindurchtreten können, werden beispielsweise 1 - 6 cm oder größere und längliche Durchbrüche eingesetzt. Auf den Stegen wiederum befinden sich kleinere runde oder quadratische Durchbrüche zum Anbringen von Produkthalterungen oder Spülmodulen.

Eine sinnvolle Voraussetzung zum Festlegen von Waschgutträgern, wie z. B. Siebkörbe sind runde, ovale oder eckige Durchbrechungen zumindest in den Ecken der Gitter oder längliche Unterteilungen von größeren Gitterdurchbrüchen. Alternativ können aber auch Erhöhungen in Ausgestaltung von Stiften das Festlegen von Siebkörben auf dem Gitter verbessern. Funktion dieser Durchbrechungen ist, insbesondere Waschkörbe über geeignete Füße festsetzen zu können oder diese so an zu ordnen, dass beim Hochklappen die Waschkörbe festgehalten werden.

In einer Weiterbildung des Gitters können zwischen den Verstrebungen andere Gitter mit kleineren Durchbrechungen und Rahmungen, wie oben beschrieben, eingesetzt sein. Denkbar wäre aber auch, dass beabstandete Haltebügel oder Abkantungen (Funktionsteile) vorhanden sind, um das Gitter besser anheben oder transportieren zu können. An diesen Funktionsteilen können auch Spülmodule befestigt werden. Weiter können Spülmodule (Spülzylinder, Spülleiste, Spülblock etc.) über die kleineren Durchbrechungen auf dem Gitter befestigt sein.

### Einsatzgebiete und Ausführungsformen

Die Erfindung wird insbesondere angewendet bei der Aufbereitung von medizinischen Instrumten sowie bei der Reinigung von Hohlrauminstrumenten in kleineren aber auch größeren RDG's (Reinigungs- und Desinfektionsgeräten) und Ultraschallbädern. Einsatzgebiete sind aber auch überall dort zu finden, wo hygienisch einwandfrei aufbereitet oder zumindest gereinigt werden muss. Sinnvolle Einsatzgebiete sind die Medizintechnik, der Pharma- und Laborbereich, aber auch der Haushaltsbereich. Insbesondere kleinere, ambulant operierende Arztpraxen und Gesundheitszentren können von der Neuerung profitieren, weil hiermit eine effektive Produktaufbereitung auch in einfachen RDG's sowie in haushaltsüblichen Geräten ermöglicht wird. Vorhandenen Maschinenwagen können mit Reinigungsvorrichtungen, Funktionssiebkörben oder -gitter aufgerüstet sein und damit die Einsatzmöglichkeiten des Wagens erweitern und/oder um damit die Reinigungsleistung des Reinigungsgerätes zu verbessern.

Dabei bieten sich folgende Ausgestaltungsformen für eine zumindest rollbaren, ausziehbare, eintauchbare oder einhängbare Reinigungsvorrichtung für RDG's oder Ultraschallbäder an:
1. Reinigungsvorrichtung mit einer zwei - oder dreiteiligen Lagerungsebene.
2. Reinigungsvorrichtung mit schräg gestellten Gitter oder Siebkörben bei der Reinigung.
3. Reinigungsvorrichtung, die so große Durchbrüche aufweist, dass hiermit Siebkörbe schräg gestellt werden können.
4. Reinigungsvorrichtung, bei der die Gitter und Siebkörbe entnehmbar sind und in eine Schublade eingebracht werden und eine Produktlagerung ermöglichen ohne die Produkte umpacken zu müssen.
5. Reinigungsvorrichtung mit einem klappbaren oder ausziehbaren Gitter, wovon zumindest ein Gitter ein Spülmodul oder eine anpressbare Rahmung trägt.
6. Reinigungsvorrichtung, die nach oben hin über die hochklappbaren Gitter mit Spülmodulen zumindest eine geöffnete Lagerungsebene zum Reinigen von langen Hohlrauminstrumenten aufweist.
7. Reinigungsvorrichtung, die in unterschiedlichen Etagen einer Reinigungsmaschine eingeschoben werden kann.
8. Reinigungsvorrichtung, die vorne und hinten in einer Reinigungsmaschine eingeschoben und herausgenommen werden kann.
9. Reinigungsvorrichtung, die ein Maschinenkorb ist und zumindest im Bodenbereich oder an einer Seitenwand eine anpressbare Rahmung oder Vertiefung oder die Maschinenkammerwand selbst eine Rahmung oder Vertiefung zur Medienweiterleitung besitzt.
10. Reinigungsvorrichtung, die ein Siebkorb ist und zumindest eine anpressbare Wandfläche, eine Rahmung oder eine Dichtung besitzt.
11. Reinigungsvorrichtung, bei der ein Siebkorb zumindest einen lösbaren Vorbau und/oder eine Behandlungseinheit (Ultraschallschwinger) besitzt
12. Reinigungsvorrichtung, die ein Maschinenkorb, ein Siebkorb oder ein Gitter ist und mit einer Ultraschallvorrichtung und/oder Maschinenausleitung kombinierbar ist.
13. Reinigungsvorrichtung, die ein Maschinenkorb, ein Siebkorb oder ein Gitter ist und zur Kennung der korrekten Kopplung diese zumindest elektronisch anzeigt.
14. Reinigungsvorrichtung nach einem der Punkte 9 - 12, die eine geeignete Fläche und/eine Rahmung oder Vertiefung beisitzt und diese mit einer Kammerwandung dichtend verbunden werden kann, wobei über die Kammerwand Medien und/oder Ultraschall applizierbar sind.

Hinsichtlich der umfangreichen Möglichkeiten bieten sich Siebkörbe und Gitter, mit folgenden Ausgestaltungsbeispielen an:
1. Gitter und Siebkörbe, die stapelbar sind und/oder eine Lagerungsebene herstellen.
2. Gitter und Siebkörbe, die Silikonpufferfüße aufweisen, die auch rollbar sein können.
3. Gitter und Siebkörbe mit einer anpressbaren Rahmung und/oder mit einem Medienmodul zur Weiterleitung von Medien.
4. Gitter und Siebkörbe mit einem Handlauf, einer Rahmung oder Bügel zum Tragen.
5. Gitter und Siebkörbe mit Silikonhalterungen (Silikonnoppenstreifen, Silikonhaltemulden etc.), um hiermit zumindest medizinische Produkte zu lagern und/oder zu fixieren.
6. Gitter und Siebkörbe, die in genormte oder bekannte Sterilcontainer oder in eine Schublade passen.
7. Gitter mit steckbaren Durchbruchselementen, damit diese so platzierbar sind, dass hiermit zumindest Siebkörbe feststellbar werden.
8. Gitter mit Erhebungen in Ausgestaltung von Stiften o. ä., damit hiermit Siebkörbe oder ähnliches festlegbar wird.
9. Gitter mit einem fest eingebauten Dreharm zur Verteilung von Reinigungsmedium.
10. Gitter mit auswechselbaren Füßen in Ausgestaltung von Silikonscheiben.
11. Gitter oder Siebkörbe, die mit einer elektronischen oder einer mechanische Kennung (Schlüssel-Schloss-Prinzip o. ä.) ausgerüstet sind, um mit einer Maschine kommunizieren zu können und/oder diese zu steuern oder eine Dokumentation der Aufbereitung ermöglicht wird.
12. Gitter und Siebkörbe mit einem lösbaren Vorbau an denen eine Ultraschallvorrichtung montiert ist oder damit eine Kammer zur Medienverteilung hergestellt wird.

Die Vorrichtungen oben beschriebener Reinigungsvorrichtungen ermöglichen unterschiedlich ausgestaltete Varianten, die beispielsweise individuell auf die benötigten Reinigungsbedingungen, änderbar sind.

Bei einer praxisrelevante Ausgestaltung eines Maschineneinsatzes, der wenigstens ein horizontal ziehbares Korbgitter o. ä. mit Rollen oder Schienen zum Ausziehen in einer Reinigungsmaschine aufweist, ist die obere Ebene eine dreiteilige Ebene, bei der jedes einzelne Gitter über ein unteres Gegenlager aufklappbar ist, damit die darunterliegenden Produkte, Siebkörbe usw. entnommen werden können.

Eine andere Ausgestaltungform wird durch einen mehrteiligen Maschineneinsatz ermöglicht, der über Schienen und/oder Rollen, wie Register oder Schubladen in unterschiedlichen Höhen in einer Reinigungskammer angeordnet werden kann.

Eine mobile Reinigungsvorrichtung wird aus einem Gitterkorb mit einer wandständigen Rahmung, die eine äußere Dichtung besitzt, gebildet. Dieser Korb wird über eine Gerätetüre eingebracht und kann über diese an die hintere Kammerwandung gedrückt werden, um eine Medienausleitungsöffnung zu überdecken. Alternativ kann der Gitterkorb eine dichtende Fläche oder eine dichtenden Rahmung oder eine Vertiefung sein und diese mit einer Maschinenkammerwand lösbar gekoppelt werden. Eine andere mobile Reinigungsvorrichtung ist ein Gitterkorb mit zumindest einer Rahmung, der eine äußere Dichtung besitzt und von oben in ein Tauchbad eintauchbar ist. Je nach Lage der Kammerausleitungsöffnung, kann die Rahmung seitlich oder bodenständig ausgeführt sein. Derartige Tauchbäder können kleine Ultraschallbäder, aber auch größere und kombinierte Tauchbäder sein. Auch hier kann alternativ die Maschinenkammer eine dichtende Rahmung besitzen, und hieran können geeignete Gitter, Gitterkörbe und/oder Maschinenwagenflächen anpressbar zu sein.

Ein hygienische Ausgestaltung wird durch eine Kammer ermöglicht, die beidseitig offen ist und die Medienzuführung zumindest seitlich und/oder von oben oder unten erfolgt. Dabei ist vorgesehen, dass der Maschineneinsatz auf einer unreinen Seite eingebracht bzw. eingeschoben und auf der reinen Seite wieder herausgeschoben werden kann. Die Medienkopplung an die Kammerwand wird beispielsweise durch seitliches anpressen erreicht.

Bei der Verwendung von Gitter ist vorgesehen, dass diese auf einen äußeren Rahmen eines einfachen Maschinenwagens oder einem Maschinengitterkorb aufliegen. Derartige Maschinenwagen bestehen aus einem Drahtgitter. Die Gitter bilden über diesen Rahmen oder Korb eine weitere zumindest teilbare Lagerungsebene aus. Durch die Art des Gitters wird die Beladungskapazität eines Maschinenwagens verbessert. Dadurch, dass viele derartige Maschinenwagen standardisiert oder ähnlich gestaltet sind, lassen sich gleichartige Gitter bei Maschinenwagen der unterschiedlichen Hersteller anwenden.

Bei einer anderen Anwendung wird ein Ultraschallbad zur Reinigung verwendet und im Bad kann ein Rahmen eingehängt sein. Auf diesem Rahmen werden die Gitter abgelegt. Dabei entsteht eine teilbare Lagerungsebene auch zur Ablage von Waschgutträgern. Es kann erforderlich sein, dass die Gitter auch schräg gestellt werden müssen. Über die Gitter können Spülmodule (Spülleisten, Spülzylinder etc.) befestigt sein, um über eine Pumpe Behandlungsmedium in Hohlraumprodukte ein- und auszuleiten. Die Pumpe kann im Ultraschallbad eingebaut oder nur zugeschaltet sein. Alternativ kann das Gitter auch Produkthalterungen besitzen und Produkte tragen. Anschließend kann zumindest das Gitter aus dem Bad entnommen und in ein RDG überführt werden, um die Behandlung zu erweitern. Mit oder ohne einen weiteren Sterilisationsschritt kann das gleiche Gitter mit Produkten entnommen und in eine Schublade eingebracht werden. Diese Art der Prozesskette ist zumindest im Dentalbereich für Dentalinstrumente, aber auch für Instrumente in der Chirurgie sinnvoll. Im Fall von daVinci Instrumenten können z. B. diese so auch vorbehandelt und anschließend über das Gitter in einem RDG zur weiteren Behandlung eingebracht und/oder weiter transportiert werden. Insofern ein Spülmodul benötigt wird, kann dieses während der gesamten Aufbereitungskette auf dem Gitter verbleiben.

Die einfachste Ausführungsform eines Gitters wird aus einem Blech hergestellt, das seitlich gebogen und beabstandete Handläufe besitzt. Als Gegenlager dient die seitliche Abkantung oder eine Abkantung mit einer ankerförmigen unteren Ausformung, damit das Gitter am umlaufenden gitterförmigen Maschineneinsatz aufklappbar ist. Zusätzlich können hier auch Silikonteile als Stopper oder Halteelemente eingesetzt sein.

Weiterführende Ausführungsvorrichtungen werden vom Gitter gebildet, die beispielsweise eine fest montierte Spülleiste oder einen Spülzylinder aufweisen, um hiermit medizinische Hohlrauminstrumente, die bevorzugt auf dem Gitter liegen, mit Reinigungsmedium zu versorgen. Alternativ kann die Spülleiste auch mobil oder fest eingebaut mit einer Siebkorbseitenwandung verbunden sein, um eine Hohlproduktreinigung zu ermöglichen. Ferner kann hier auch vorgesehen sein, dass die Gitter bei der Aufbereitung schräg gestellt werden, um besonders lange Instrumente aufzunehmen oder ein besseres Abtropfen von Reinigungsmedium zu ermöglichen. Letzteres verbessert z. B. auch die Instrumententrocknung.

### Ausführungsbeispiele

Im folgendem wird die Erfindung anhand von Ausführungsbeispielen im Zusammenhang mit der Zeichnung ausführlicher erläutert.
- Fig. 1: zeigt in einer Schnittdarstellung die Neuerung in Ausgestaltung eines Siebkorbs mit einem festlegbaren Vorbau.
- Fig. 2: zeigt in Seitansicht eine Maschinenkammer einer Reinigungsmaschine mit einer vorderen einschiebbaren hinteren Kopplung.
- Fig. 3: zeigt in Seitenansicht ein Ultraschallbad eines Ultraschallgerätes mit einem tauchbaren Gitterkorb mit einer bodenständigen Kopplung.
- Fig. 4: zeigt in einer Seitansicht eine Maschinenkammer einer Reinigungsmaschine mit einer einschiebbaren seitlichen Kopplung.
- Fig. 5: zeigt im Schnitt einen neuartigen Siebkorb mit einer seitlichen Rahmung oder Vertiefung.
- Fig. 6: zeigt eine Reinigungsvorrichtung mit einer Rahmung mit einem Ultraschallerzeuger, der an ein Siebkorb eingebracht ist.
- Fig. 7: zeigt einen neuartigen Siebkorb bzw. eine Reinigungsvorrichtung mit einer dichtenden Rahmung und/oder einer dichtenden Fläche
- Fig. 8: zeigt eine Siebkorb-Reinigungsvorrichtung bei der Ultraschall und Reinigungsmedium seitlich eingeleitet wird.
- Fig. 9: zeigt in Draufsicht einen Maschinenwagen mit einer geteilten Funktionsebene und unterteilten Medienverteilerrohren.
- Fig. 10: zeigt eine Reinigungsvorrichtung als Gitter in Draufsicht mit Durchbrüchen zum Festlegen von Waschträgern.
- Fig. 12: zeigt ein Gitter in Draufsicht mit Durchbrüchen zum Festlegen von Waschträgern mit mehreren länglichen großen Durchbrüchen.
- Fig. 13: zeigt ein Gitter in Draufsicht mit Durchbrüchen zum Festlegen mit einem feinmaschigen Gitter.
- Fig. 14: zeigt eine Reinigungsvorrichtung mit einem Gitter in Draufsicht und einem Dreharm.

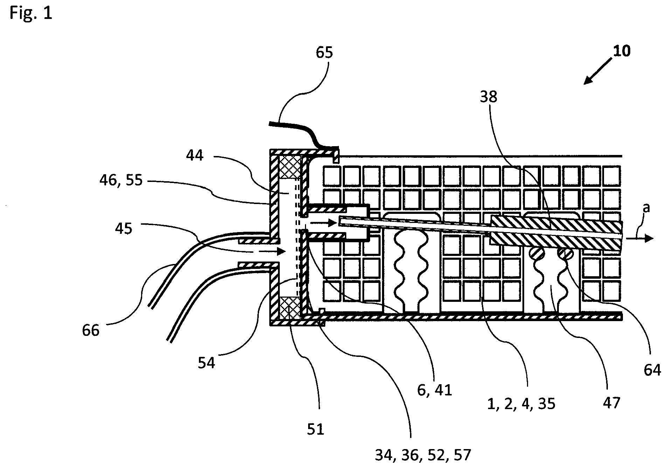

Figur 1 zeigt in einer Schnittdarstellung die Neuerung in Ausgestaltung eines Siebkorbs mit einem festlegbaren Vorbau. Die gezeigte Neuerung **10** zeigt einen Siebkorb **35** in Verbindung mit einem Vorbau **55.** Anstelle eines Siebkorbs **35** kann hier ein Gitterkorb **4** bzw. ein Gitter **2** oder ein Maschineneinsatz **1** vorhanden sein. Dabei wird eine Kammer **44** durch Anbringen eines lösbaren Vorbaus **55** in Verbindung mit einer vorzugsweise planen und koppelbaren Siebkorbseitenwand **52** hergestellt. Alternativ kann die plane Siebkorb Kopplungsfläche **52** auch an eine Kammerwand **46** mit einer Dichtung **51** angedrückt sein, um eine dichtende Kammer **44** herzustellen. Anstelle der planen Kopplungsfläche **52** kann diese eine flächige Rahmung **36** oder eine flächige Vertiefung **34** aufweisen bzw. von einer umlaufende Nute **57** umrahmt sein und zumindest eine innenliegende Öffnung **6** besitzen. Die hergestellte Kammer **44** wird über einen Medienanschluss **45,** der beispielsweise über eine Zuführleitung **66** (Schlauchleitung) in einer Reinigungsmaschine angeschlossen ist, mit Reinigungsmedium gefüllt. Der Medienanschluss **45** kann hier auch einen steckbaren Adapter oder Maschinenanschluss aufweisen. Alternativ oder zusätzlich kann hier auch ein Ultraschallschwinger vorhanden sein. Der Durchtritt von Reinigungsmedium ist über die **Pfeile a** angedeutet. Die Kammer **44** kann einen innenliegenden Filter **54** aufweisen. Um den Filter **54** entfernen zu können ist der Vorbau **55** über eine Federelement bzw. Hebel **65** lösbar. Hier kann alternativ vorgesehen sein, dass der Vorbau über eine Manschette oder ähnliches mit dem Siebkorb verbunden wird. Der Vorbau kann dabei selbst ganz aus Silikon hergestellt sein, um durch die Elastizität des Vorbaus festlegbar ist. Der Abstand zur Siebkorbwand **52** wird hier über eine umlaufende rahmende Dichtung **51** hergestellt. Eingeleitetes Medium wird zumindest über eine innenliegende Austrittsöffnung **6** in der ein Adapter oder ähnliches eingebracht ist, weitergeleitet. Anstelle eines Anschlussadapters kann hier auch eine selbstverschließbare Kappe (Kappenadapter) oder ein Ventil **41** vorhanden sein. Wie hier gezeigt, befindet sich ein Schafft eines medizinischen Hohlrauminstrumentes **38** in dem Kappenadapter. Zur besseren Lagerung kann das Instrument **38** über Silikonringe **64**, die über biegbare Siebkorbformteile **47** gehalten werden, fixiert oder gelagert sein. Damit wurde aus einem Siebkorb eine vielseitig einsetzbares und änderbares Spülmodul bzw. eine Reinigungsvorrichtung zur Reinigung von Hohlrauminstrumenten.

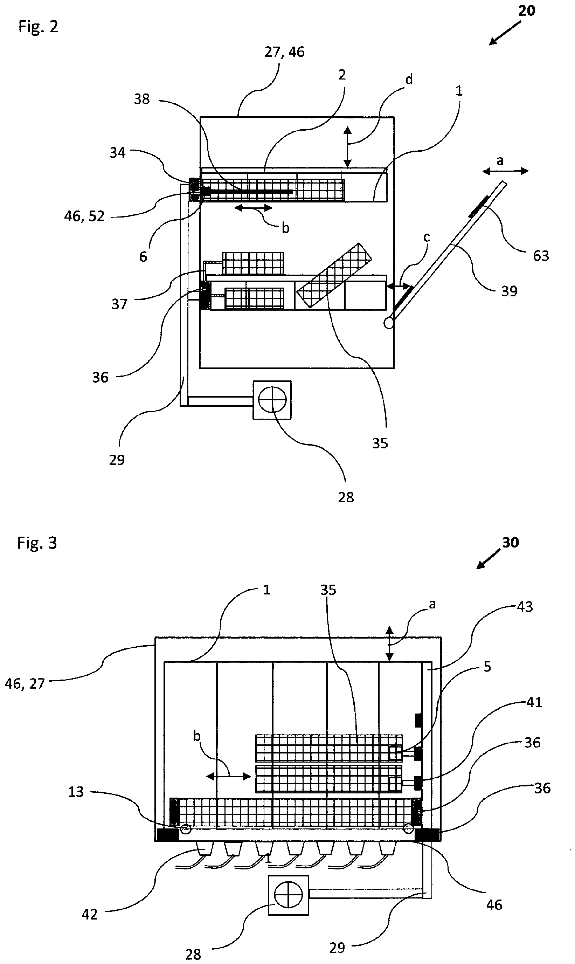

Figur 2 zeigt in einer Seitansicht eine Maschinenkammer eines Reinigungsmaschinemit mit einer vorderen einschiebbaren hinteren Kopplung. Die Darstellung zeigt ein Reinigungsgerätes **20** mit einer Maschinenkammer **27** und beinhaltet einen unten und einen oben angeordneten einschiebbaren Gitterkorb **1** bzw. Maschinenwagen (dargestellt durch **Pfeil b**) mit einem oberen aufliegenden Gitter **2** zur Herstellung einer weiteren Lagerungsebene. Dieses Gitter **2** kann teilbar sein und ist vorzugsweise hochkappbar (durch **Pfeil d** angedeutet), ohne hier aufliegende Produkte, wie z. B. einen Siebkorb **35** etc., entnehmen zu müssen. Große Durchbrüche im Gitter sorgen auch dafür, dass Siebkörbe **35** hiermit schräg gestellt werden können. Um z. B. den unteren Maschinenwagen an eine Medien- oder Ultraschallversorgung anzuschließen, besitzt dieser eine Rahmung **36** mit einer abgehenden Medienleitung **37.** Hingegen besitzt der obere Maschinenwagen oder Gitterkorb nur eine Fläche **52** mit zumindest einem Anschlussadapter **6**, um mit einer Vertiefung **34** bzw. Mulde in der Maschinenkammerrückwand **46** eine Spül- bzw. eine Reinigungsvorrichtung auszubilden. Alternativ kann zumindest die gesamte hintere Kammerwand **46**, doppelwandig sein, um Medien flächig ausleiten zu können. In die Doppelwand können ferner Heizstäbe zum flächigen Erhitzten von Medien eingesetzt sein. Durch die hergestellte Medienverbindung kann ein Hohlrauminstrument **38** angeschlossen und durchspült werden. Der Medienausstrom wird über eine Pumpe **28** und einer Medienleitung **29** ermöglicht. Damit beide Maschinenkörbe sicher mit der hinteren Kammerwand zumindest einen Medieneinstrom herstellen können, besitzt das Reinigungsgerät eine auf- und zu klappbare Tür **39** (dargestellt durch **Pfeil a**) mit einer vorzugsweise elastischen oder zusammenpressbaren Anpressflächen **63.** Wird die Klappe oder die Türe **39** geschlossen, so werden zumindest die Gitterkörbe **1** über die Anpressfläche **63** an die hintere Kammerwandung gedrückt (durch **Pfeil c** dargestellt).

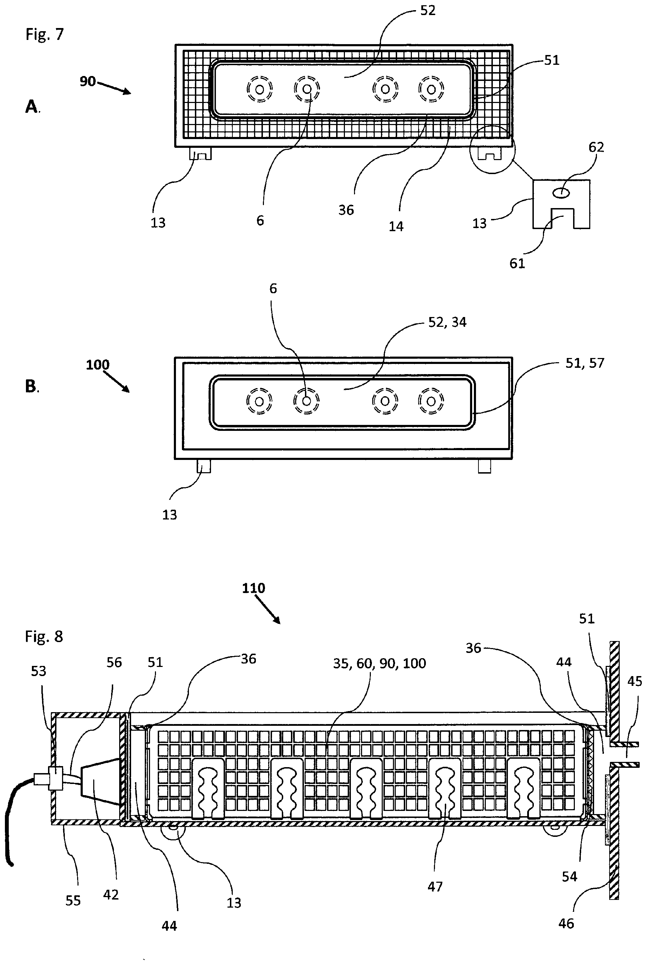

Figur 3 zeigt in Seitenansicht ein Ultraschallbad eines Ultraschallgerätes mit einem tauchbaren Gitterkorb mit einer bodenständigen Kopplung. Es ist ein Ultraschallgerät **30** mit einer Ultraschallkammer **27** und einem eingetauchten Gitterkorb **1** gezeigt. Der Gitterkorb **1** besitzt zumindest einen seitlich und flächig ausgebildeten Medienraum (z. B. rechteckig) oder eine große Rohrleitung **43** mit abgehenden selbstverschließbaren Medienanschlüssen **41.** An diese können über Siebkörbe **35** mit einer Steckkupplung, die sich an Medienverteilerblöcke, Spülleisten **5** etc. angeschlossen sind, Medien ausgeleitet und/oder für Reinigungszwecke verteilt werden. Der untere Siebkörbe **35** mit beabstandeten Rahmungen **36** stellt eine Reinigungsvorrichtung dar (siehe Figur 7), die vorzugsweise über Scheiben oder Rollen **13** festlegbar ist. Dabei können die oberen Siebkörbe durch das Einschieben der Steckverbindung in den Medienanschluss **41** festlegbar sein (dargestellt durch **Pfeil b**). Die Neuerung ermöglicht jedoch, wie hier gezeigt, einen sicheren Hauptmedienanschluss in der Form, dass der Gitterkorb **1** nach unten hin eine flächige Umrahmung **36** am Gitterkorb besitzt, die eine Öffnung am Kammerboden **46** überdeckt, um Medien weiterleiten zu können. Neu dabei ist, dass alleine die Schwerkraft des Gitterkorbs **1** ausreicht, um eine Medienausleitung von einer Pumpe **28** ausgehend und über Zuleitungen **29** in eine Umrahmung **36** zu ermöglichen und damit eine Reinigungsvorrichtung, um Produktkanäle zu durchströmen. Dabei braucht die Kopplung nicht exakt zu sein, was wesentlich ist, um Fehlfunktionen zu vermeiden, wenn die Gitterkörbe **1** herabgesenkt werden. Zumindest von unten erfolgt eine Ultraschallbehandlung über Ultraschallschwinger **42.** Eine Medienweiterleitung in einem Siebkorb **35** hingegen kann ebenfalls durch eine seitliche Rahmung **36** erfolgen. Dies ist möglich, wenn der Siebkorb **35** mit einer beidseitigen beabstandeten Seitenrahmung **36** festgelegt ist und dadurch an einen seitlich zumindest flächig ausgebildeten Medienraum **43,** der auch die ganze hintere Wand einnehmen kann, angedrückt wird, um einen Medieneinstrom herzustellen.

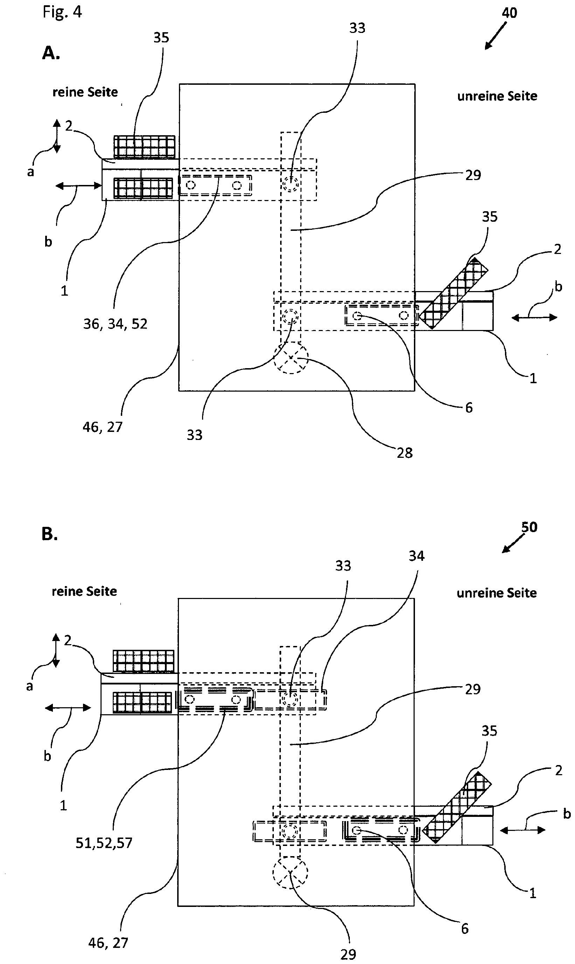

Figur 4 zeigt in einer Seitenansicht eine Maschinenkammer einer Reinigungsmaschine mit einer einschiebbaren seitlichen Kopplung. **A** zeigt in Abbildung **40** einer Reinigungsmaschine die gegenüber Figur **1** im Aufbau gleich ist, jedoch Gitter- oder Maschinenkörbe **1** bzw. Maschinenwagen besitzt, die in unterschiedlicher Höhe beidseitig (reine und unreine Seite) einschiebbar (skizziert durch **Pfeil b**) sind. Zur Überdeckung mit einer Kammeröffnung **33** (Medienöffnung) besitzt der Gitterkorb **1** eine vorstehende Rahmung **36,** eine Vertiefung **34** oder eine Wandfläche **52** mit zumindest 2 x hinteren Adaptern **6** oder ähnlichen Anschlüssen. Durch das Einschieben eines Gitterkorbs bzw. Maschinenwagens **1** wird eine seitliche Überdeckung mit einer Kammerwand **46** der Reinigungsmaschinenkammer **27** erreicht. Vorzugsweise mit einer beabstandeten gegenüberliegenden Anpressvorrichtung wird die Rahmung **36** oder eine Vertiefung **34** des Gitterkorbs **1** an die Kammerwand **46** gedrückt. Die Anpressvorrichtung kann mechanisch (über eine Feder etc.) oder durch einen elektronisch auslösbaren Anpressstempel oder ähnlichem unterstützt sein. Um die Beladekapazität zu erhöhen sowie die Handhabung zu verbessern, kann der Gitterkorb **1** zumindest ein Gitter **2** aufweisen (siehe hierzu Beschreibung Fig. 1). **B** zeigt in Abbildung **50** ebenfalls eine Reinigungsmaschine mit beidseitigen einschiebbaren Gitter- oder Maschinenkörben **1.** Hier jedoch besitzt die Maschinenkammerwand 46 seitliche Vertiefungen **34** mit einer mittig angeordneten Medienöffnung **33.** Der Maschinenkorb besitzt hingegen eine Kopplungsfläche **52** mit einer umlaufenden Nute (Umrahmung) **57.** Mit der Kopplungsfläche **52** wird eine Überdeckung mit der Vertiefung **34** in der Kammer **27** hergestellt. In diese ist vorzugsweise eine Dichtung **51** eingelassen. In **A** und **B** erfolgt der Medientransport über eine Pumpe **28** in Verbindung mit Rohrleitungen **29.** In **A** kann die Pressung an die Kammerwand **46** mechanisch unterstützt sein. Eine Medienverteilung wird über eine Spülverteilerfläche am Gitterkorb 1 mit hier vorhandenen Adaptern **67** ermöglicht.

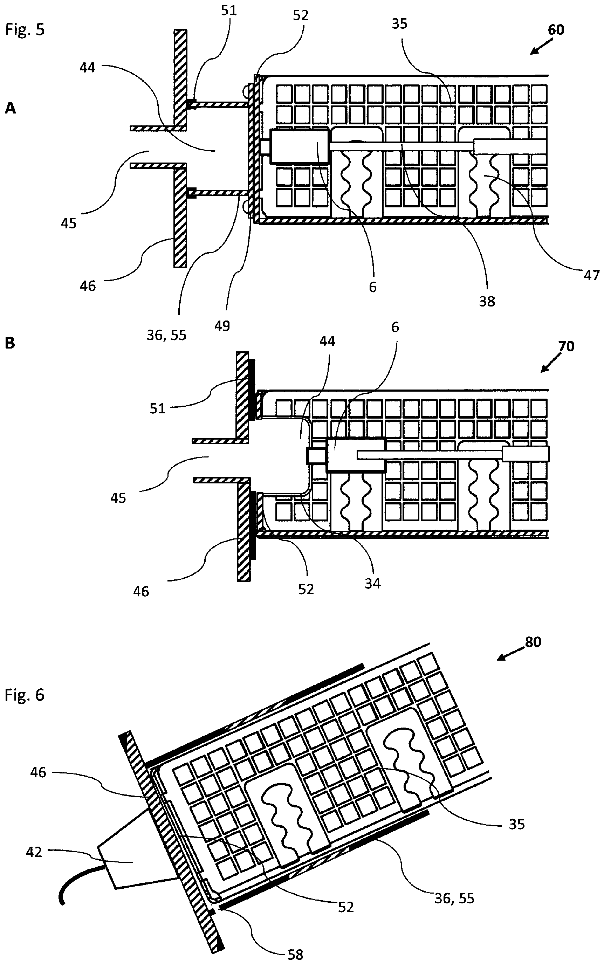

Figur 5 zeigt im Schnitt einen neuartigen Siebkorb mit einer seitlichen Rahmung oder Vertiefung. Die Abbildung **60** zeigt in **A** einen neuartigen Siebkorb mit seitlichen Formteilen **47** über eine Platte **49** vorderseitig angebrachten Rahmung **36** und stellt selbst eine Reinigungsvorrichtung dar oder bildet diese aus. Die Körbe **50** können aber auch über die Rahmung z. B. eine verschiebbare Lagerungsebene ausbilden. Die Rahmung **36** besitzt vorne eine umlaufende Dichtung **51.** Nach Innen ist ein Instrumentenkappenadapter 6 angeordnet, in dem ein medizinisches Hohlrauminstrument **38** eingebracht ist. Um Reinigungsmedium über eine Kammerwand **46** mit einer Medienzuleitung **45** zu Reinigungszwecken einleiten zu können, muss der Korb über die Rahmung an die Kammerwand **46** gedrückt werden. Dadurch entsteht mit der Rahmung **36** und der Kammerwandung **46** eine flächige Kammer **44** bzw. eine gekoppelte Reinigungsvorrichtung. Über diese Kammer **44** wird Medium in die Adapter 6 verteilt. Die so hergestellte Behandlungsvorrichtung zur Reinigung von Hohlraumprodukten ist kostengünstiger und platzsparender als der Einsatz von separaten Spülleisten oder vergleichbare Spülmodule. In **B** ist ein Siebkorb **70** mit einer nach innen gerichteten Vertiefung bzw. Halterungsmulde **34** gezeigt. Diese besitzt zumindest einen Anschlusskappenadapter **6** und ist damit auch eine Reinigungsvorrichtung für Hohlrauminstrumente. Wie in Abbildung **A**, bildet der Siebkorb **70** mit einer Kammerwand **46** über ein flächigem Dichtelement **51** eine Kammer **44** aus, über die Medien in ein Hohlrauminstrumente **38** eingeleitet werden kann. Sinnvollerweise befinden sich in der Vertiefung/Mulde **34** mehrere gleiche oder unterschiedliche Anschlussadapter **6**, um Hohlrauminstrumente durchspülen zu können. Eine flächige Dichtung vereinfacht die Herstellung einer dichtenden Kopplung. Alternativ kann die Dichtung **51** auch selbst eine Rahmung und damit eine Kammer bilden.

Figur 6 zeigt eine Reinigungsvorrichtung mit einer Rahmung mit einem Ultraschallerzeuger, bei dem ein Siebkorb eingebracht ist. Die Reinigungsvorrichtung **80** kann beispielsweise ein Vorrichtungsteil oder eine selbständige Reinigungsvorrichtung in einer Reinigungsmaschine sein. Dabei kann ein Ultraschallschwinger **42** an einer Kammerrückwand **46** angebracht sein und an der Vorderseite eine großflächige Rahmung **36** bzw. einen Vorbau **55** aufweisen, in die ein Siebkorb **35** eingebracht ist und dabei eine Siebkorbwand **52** an eine Kammerwand **46** anliegt. Füllt sich der Vorbau **55** mit Reinigungsmedium über einen Dreharm, so kann der Schall effektiv übertragen werden. Über eine Auslassöffnung **58** kann langsam das eingebrachte Medium wieder ablaufen. Die Reinigungsvorrichtung **80** ermöglicht somit, dass in den unterschiedlichen Bereichen einer Reinigungsmaschine Ultraschall zum Reinigen von Produkten einsetzbar ist und gleichzeitig eine zusätzliche Außenreinigung über den Dreharm einer Reinigungsmaschine erfolgen kann.

Figur 7 zeigt einen neuartigen Siebkorb bzw. eine Reinigungsvorrichtung mit einer dichtenden Rahmung und/oder einer dichtenden Fläche. In **A** besitzt der Siebkorb **90** zumindest an einer Seitenwand eine Rahmung **36** die innenliegend in der Fläche angeordnete Austrittsöffnungen oder Anschlussadapter **6** (Luer Lock Adapter, Kappenadapter etc.) hat. Alternativ kann der Siebkorb **90** aber auch ein Maschinenkorb oder ein Gitter sein. Ferner besitzt der Rahmen **36** eine Dichtung **51** und bildet so eine offene Kammer aus mit einer hinteren Fläche **52** aus. Am Boden kann der Siebkorb **90** Pufferfüße **13** oder Festlegelemente aufweisen. Vorderseitig kann der Siebkorb **90** ein Siebkorbgitter **14** besitzen. Ferner ist ein vergrö-ßertes Fußelement **13** in Ausgestaltung eines festlegebaren Teils ausgestaltet. Das Element besitzt einen Durchbruch **62** zum Befestigen an einem Formelement im Siebkorbboden und eine Materialausformung **61** zum Festlegen auf einem Gitter, einer Rahmung etc. Die Materialausformung **61** kann auch anders gestaltet sein. Derartig gestaltete Siebkörbe ermöglichen über die Formelemente eine beabstandete und teilbare bzw. zusätzliche Lagerungsebene, die über eine untere Lagerungsebene liegt. In **B** besitzt der Siebkorb **100**, der auch ein Maschinenkorb oder ein Gitter sein kann, eine vordere Fläche **52** und/oder eine Vertiefung **34** mit hier angeordneten Adaptern **6.** Dabei kann die Vertiefung bzw. die Rahmung auch nur eine Nute **57** mit oder ohne eine Dichtung sein. Die vordere Vertiefung **34** (Mulde) ermöglicht wie bei einer Rahmung **36** eine optimale Verteilung von eingeleiteten Reinigungsmedien, wenn diese überdeckt ist und sich in der der Vertiefung **34** Spül- oder Anschlussadapter **6** befinden. Beide Siebkörbe **90** und **100** können mit einer Kammerwandung oder mit einer Maschinenwagenwandung eine Spülvorrichtung bzw. eine Reinigungsvorrichtung für die Medienweiterleitung herstellen.

Figur 8 zeigt eine Siebkorb-Reinigungsvorrichtung, bei dem Ultraschall und Reinigungsmedium seitlich eingeleitet wird. Die Reinigungsvorrichtung **110** ist ein Siebkorb **35** bzw. in Ausgestaltung einen der vorherigen Siebkörbe **35**, **60**, **90**, **100** gezeigt, die beispielsweise eine beabstandete Rahmung **36** links und rechts mit einer Kammer **44** besitzen und aus der Vorrichtung **110** entnommen werden können. Von der linken Seite kann ein Ultraschallschwinger **42** seitlich angekoppelt sein. Diese Siebkorbseite sollte vorzugsweise viele Durchbrüche zum Durchtritt des Ultraschalls aufweisen. Der Vorbau **55** bildet in der Verlängerung ein Bad aus, um hiermit Flüssigkeit zumindest zeitweise aufzufangen bzw. zu stauen. Die vorzugsweise flächige Dichtung **51**, verbessert die Dichtigkeit mit einer Kammerwandung **46.** Diese Vorrichtung wäre auch außerhalb von Reinigungsmaschinen, z. B. in Wasserbäder, einsetzbar und kann darüber hinaus auch eine funktionelle Lagerungsebene oder -Etage in einer Reinigungsmaschine herstellen. Hierfür ist das Kabelanschlusselement **53** für die Stromversorgung über Kabel **56** entsprechend gesichert. Der Siebkorb besitzt Silikonscheiben **13** zur Lagerung oder Festlegung sowie biegbare Funktionsteile **47** an der Seitenwand. An einer gegenüberliegenden Wandung des Siebkorbes kann der Siebkorb mit einer Maschinenkammerwand **46** eine abgeschlossene Kammer **44** bilden. Zur Filterung des Reinigungsmediums, das über eine Austrittsöffnung bzw. eine Medienzuleitung **45** in eine Kammer **44** eingeleitet wird, befindet sich ein austauschbarer Filter **54.** Zum Anschluss für Hohlrauminstrumente können an der Siebkorbseitenwand Adapter etc. vorhanden sein. Damit wird das weitergeleitete Medium flächig gefiltert, was einen zu hohen Druckverlust vermeiden hilft und darüber hinaus auch die Filterleistung verbessert. Derartige Filter **54** sind sinnvoll bei der Reinigung von empfindlichen und englumigen Hohlrauminstrumenten (z. B. Augeninstrumente). Damit ermöglicht die Siebkorb-Reinigungsvorrichtung **110** zusammen eine dreharmgesteuerte Reinigung, eine Ultraschallreinigung und eine Hohlraumproduktreinigung. Die eingelagerten Hohlraumprodukte müssen nicht mehr umgepackt sowie auch nicht mehr an separate Spülmodule (Spülleisten etc.) angeschlossen werden.

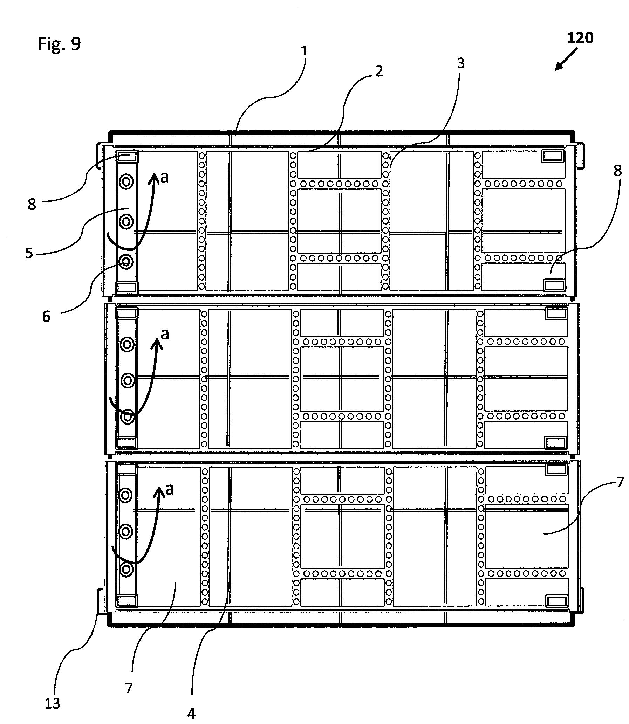

Figur 9 zeigt in Draufsicht einen Maschinenwagen mit einer geteilten Funktionsebene und unterteilte Medienverteilerrohre. Der Maschinenwagen **120** besteht aus einem Gitterkorb **1** mit einer unteren Lagerungsebene in Ausgestaltung eines großen Gitters **4**. Damit der Maschinenwagen **120** ausziehbar ist, besitzt dieser unten angebrachte seitliche Rollen **13.** Alternativ kann der Maschinengitterkorb **1** auch einhängbar sein. Auf dem umlaufenen Rahmen des Maschinenwagens **120** befindet sich eine weitere obere Lagerungsebene, die aus drei einzelnen Gitter **2** hergestellt ist. Die Gitter **2** besitzen größere Durchbrüche **7** und Querverstrebungen **3** mit Durchbrüchen. Zumindest an den Ecken des Gitters sind Durchbrüche **8** (die auch unterschiedlich groß sein können) vorhanden, um hiermit z. B. Waschträger festlegen zu können. Dabei können zumindest in den Ecken dieser Gitter kleinere Durchbrüche **8** oder Erhebungen vorhanden sein, damit auf diesen eine zusätzliche Feststellung von Waschkörben möglich ist. Alternativ können hieran auch Spülmodule befestigt oder lösbar angebracht werden. Die hier eingesetzten Gitter **2** besitzen jeweils ein Spülrohr **5** oder ähnliches mit geeigneten Adaptern **6** zum Durchspülen von Hohlrauminstrumenten. Die Spülrohre können am unteren großen Gitter **4** auch eingeschweißt sein. Die großen Durchbrüche **7** ermöglichen so auch ein Hindurchgreifen in die untere Ebene. Ein Hindurchgreifen ist dann sinnvoll, wenn auch unten Waschkörbe oder ähnliches liegen und auch hier Hohlrauminstrumente zum Durchspülen angeschlossen werden müssen. Dabei ist es möglich, die Gitter **2** individuell über zusätzliche Komponenten wie Bügel auch versetzt zueinander schräg zu stellen. Diese Möglichkeit wird durch die **Pfeile a** angedeutet. Gerade diese Ausgestaltung verbessert die Aufbereitung von medizinischen Instrumenten in kleinen Reinigungsmaschinen erheblich, weil hier der Platzbedarf beschränkt, aber auch die Reinigungskraft über die unteren und oberen Dreharme begrenzt ist.

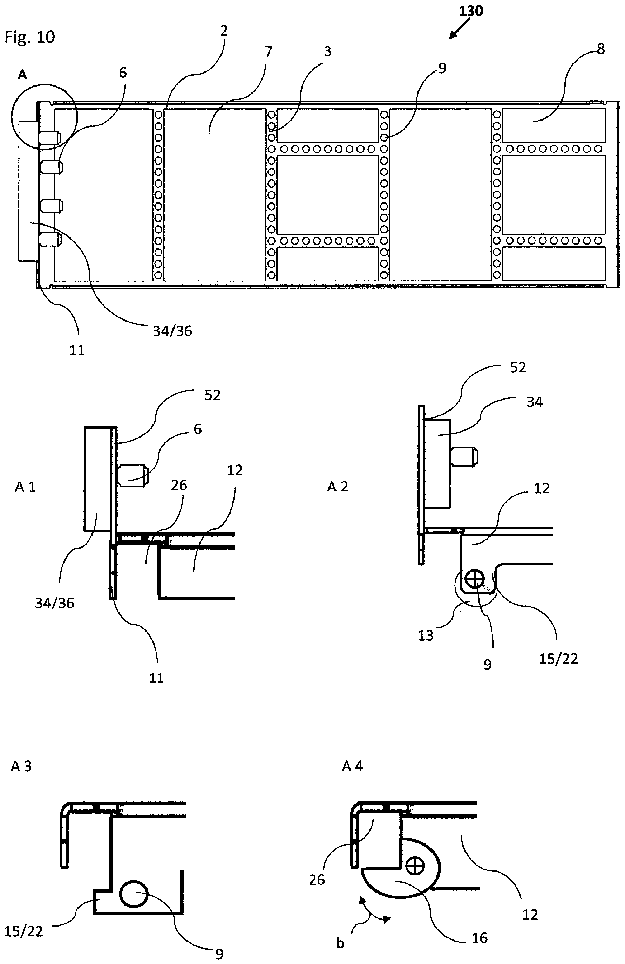

Figur 10 zeigt eine Reinigungsvorrichtung als Gitter in Draufsicht mit Durchbrüchen zum Festlegen von Waschträgern. Die Reinigungsvorrichtung **130** in Ausgestaltung eines Gitters **2** besitzt große Durchbrüche **7**, die wiederum in kleinere Durchbrüche **8** unterteilt sein können. Um Medienanschlüsse zu ermöglichen, besitzt das Gitter an einer Abkantung **11** eine flächige Rahmung **36** oder eine Vertiefung **34** mit Anschlussadapter **6.** Überdeckt das Gitter **2** eine Medienaustrittsöffnung (beispielsweise die eines Gitterwagens etc.), so kann Medium zur Reinigung weiter geleitet werden. Eine Unterteilung in kleinere und größere Durchbruchsbereiche ist sinnvoll, weil hiermit Bereiche auf dem Gitter zur Festlegung von Waschkörben (Siebkörbe), Waschbehältern usw. entstehen. Dabei ist vorgesehen, dass die Bereiche **8** so gewählt sind, dass unterschiedlich große Waschträger festlegbar sind. Auf den Querverstrebungen **3** ist eine Lochstruktur **9** angeordnet, damit hieran Produkthalterungen oder Spülmodule fixierbar sind. Beabstandete Abkantungen **11** der Gitter **2** ermöglichen eine sichere Anordnung der Gitter auf einem Maschinenwagen oder zur Ablage auf einem einhängbaren Rahmen und ermöglichen somit das sichere Hochklappen.

Der Ausschnitt **A1** zeigt eine seitliche Darstellung der Reinigungsvorrichtung in Ausgestaltung eines Gitters. Die Abkantungen **12** in Längsrichtung und die Abkantung **11** ermöglichen ein sicheres Ablegen des offenen Gitterbereiches **26** auf einer umlaufenden Rahmenstruktur eines Maschinenwagens. An der verlängerten Abkantung **11** ist eine Rahmung **36** vorhanden und in der umrahmten Fläche befinden sich mehrere Anschlussadapter **6. A2** zeigt ebenfalls eine Reinigungsvorrichtung mit einem vergleichbaren Aufbau wie **A1** mit jedoch einer Vertiefung **34** und hier enthaltenden Adaptern. Die Vorrichtung besitzt jedoch zusätzlich eine erhöhte Fußstruktur **15** bzw. **22.** Über einen Durchbruch **9** kann hier eine Scheibe **13** oder eine Rolle o. ä. angebracht werden. Diese Ausgestaltung ermöglicht auch die Stapelung der Vorrichtung untereinander. Auch hier sind die Seitenwände des Gitters **12** abgewinkelt. **A3** skizziert einen Ausschnitt eines reinen Gitters mit einem ankerförmigen Fuß **15** bzw. **22.** Über den Durchbruch **9** können z. B. Scheiben zum Rollen des Gitters oder zur besseren Stabilisierung vorhanden sein. **A4** stellt eine Weiterentwicklung eines Gitters mit der Möglichkeit dar, diese mit einem Rahmen festlegbar über eine drehbare (dargestellt durch den **Pfeil b**) ovale Scheibe oder über einen drehbaren Haken **16** oder ähnliches zu ermöglichen. Dabei wird der offene Gitterbereich **26** auf einem Rahmen abgelegt und anschließend über einen Haken **16**, der auch eine Feder zur besseren Feststellung besitzen kann, festgelegt.

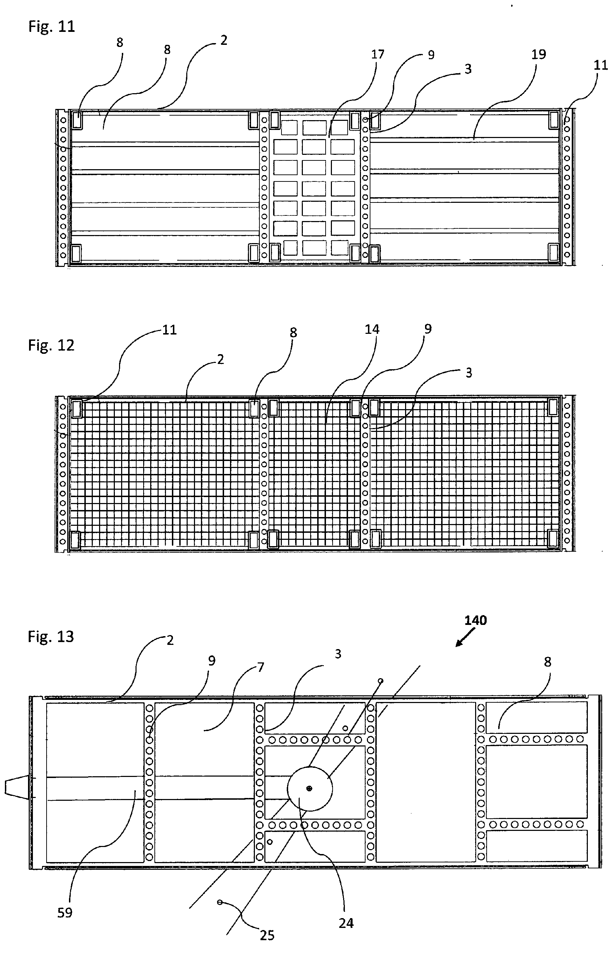

Figur 11 zeigt ein Gitter in Draufsicht mit Durchbrüchen zum Festlegen von Waschträgern mit mehreren länglichen großen Durchbrüchen. Das Gitter **2** besitzt beabstandete U-förmige seitliche Abkantungen **11** zur Ablage auf einem Rahmen. Zum Festlegen von Waschgutträgern (z. B. Siebkörbe) wurden die großen Durchbrüche über Stege **19** in kleinere Bereich **8** unterteilt. Diese parallelverlaufenden Durchbrüche **8** haben darüber hinaus noch den Vorteil, dass hiermit flache Gestelle oder Instrumente schräg gestellt werden können. Der zentrale Bereich des Gitters besitzt ein rechteckiges Durchbruchsgitter **17** mit Stegen **19.** Zur Ablage oder zum Festlegen von kleinen Waschgutträgern sind parallelverlaufende oder kleinere Durchbrüche **8** vorgesehen. Die Querverstrebungen **3** besitzen Durchbrüche **9** zur Erweiterung der Befestigungsstrukturen auf dem Gitter.

Figur 12 zeigt ein Gitter in Draufsicht mit Durchbrüchen zum Festlegen und ein feinmaschiges Gitter. Auch hier ist ein Gitter **2** bzw. ein Lagerungsgitter dargestellt, dass eine feinmaschige Lagerungsebene **14** besitzt. Hier kann vorgesehen sein, dass die feinmaschige Lagerungsebene **14** herausnehmbar und/oder teilbar ist und durch andere Gitternetze ersetzt werden kann. Als Festlegpositionen für weitere Waschträger sind rechteckige Durchbrüche **8** vorgesehen. Diese können aber auch zum Festlegen von Produkthalterungen eines und/oder von mehreren für sich stehende feinmaschige Gitter **14** ausgestaltet sein. Derartige Gitter **2** stellen selber eine Ablage-Ebene für Produkte oder medizinische Instrumente dar und können eine erhöhte umlaufende Wandung aufweisen. Auf den Querverstrebungen **3** mit den Durchbrechungen **9** oder auf dem feinmaschigen Gitter **14** können hierzu Silikonnoppenbänder oder ähnliches angebracht sein. Derartige Gitter sind damit auch zur Lagerung in einer Schublade oder zum Transport von Waschgütern geeignet. Alternativ kann anstelle des Gitters auch ein Korb die Aufgabe übernehmen.

Figur 13 zeigt eine Reinigungsvorrichtung mit einem Gitter in Draufsicht und einem Dreharm. Die dargestellte Reinigungsvorrichtung **140** besteht aus einem Gitter **2** auf dem ein steckbarer Dreharm **24** mit Medienaustrittsöffnungen **25** angebracht ist. Dieser Dreharm **25** ist unter dem Gitter angebracht. Über ein steckbares Rohrteil **59** wird der Dreharm in einer Reinigungsmaschine mit Reinigungsmedium versorgt. Alternativ kann die Reinigungsvorrichtung **140** auch in geeignete Ultraschallbäder eingebracht sein, um eine Mediendurchmischung durch Drehen herbeizuführen. Anstelle des Dreharms kann auch ein Spülmodul vorhanden sein, um Medien in Hohlrauminstrumente einzubringen. Diese Ausgestaltung verändert bzw. verbessert das Reinigungsergebnis hierüber- und hierunterliegender Waschgüter.

### Wichtige Zeichnungserklärungen

- 1: Maschinengitter, Maschineneinsatz oder Gitterkorb
- 2: Gitter oder Lagerungsgitter
- 4: großes Gitter oder großer Gitterkorb
- 5: Spülmodul oder Spülrohr
- 6: Austrittsöffnung, Adapter, Anschluss- oder Kappenadapter
- 8: Durchbrüche zum Festlegen von Waschgutträgern
- 13: Rollen, Scheiben oder Festlegelemente
- 14: feinmaschiges Gitter oder Siebkorbgitter
- 16: Haken
- 24: Dreharm
- 27: Maschinenkammer
- 33: Kammer- oder Medienöffnung
- 34: vertiefte Vorrichtungsfläche mit zumindest einer Austrittsöffnung
- 35: Siebkorb oder Waschgutträger mit oder ohne Spülmodul oder Spülfunktion
- 36: Umrahmte Fläche oder Rahmen mit zumindest einer Austrittsöffnung
- 39: Türe oder Klappe
- 41: selbstverschließbarer Medienanschluss, Ventil
- 42: Ultraschallschwinger
- 43: seitliche Medienkammer oder Medienrohr
- 44: Kammer
- 45: Medienzuführleitung
- 46: Kammerwand
- 52: koppelbare Vorrichtungsfläche mit zumindest einer Austrittsöffnung, vorzugsweise plan
- 51: Dichtung
- 54: Filter
- 55: Kopplungsteil, Vorbau
- 57: Nute die eine Fläche mit zumindest einer Austrittsöffnung umrahmt
- 59: steckbares Rohrteil
- 61: Materialausformung zum Festlegen eines Siebkorbs oder Gitters

## Patentansprüche

1. Reinigungsvorrichtung für Reinigungsgeräte, bei der zumindest ein Maschineneinsatz, eines Einschubwagen, ein Gitter, ein Gitterkorb oder ein Siebkorb oder eine Kombination aus diesen zum Einsatz kommt, **dadurch gekennzeichnet, dass** die Vorrichtung erst eine Medien befüllbare Doppelwandung (34, 36, 46, 52, 57) durch eine Überdeckung mit einer beabstandeten Kammerwand (46, 34, 36) oder mit einem Vorbau (55) oder Rahmung (36, 51, 57) ausbildet, um über Austrittsöffnungen (6, 41) und/oder einem Ultraschallschwinger (42) Medium und/oder den Ultraschall für die Reinigung von Produkten zu verteilen.

2. Reinigungsvorrichtung nach Anspruch 1, **dadurch gekennzeichnet, dass** eine umrahmte (36, 51, 57) oder eine vertiefte Fläche (34) und/oder nur eine Fläche (52) mit zumindest einer vorhandenen Austrittsöffnung (6, 41) eine Kammer (44) durch Anpressung über die Gewichtskraft der Reinigungsvorrichtung, durch mechanische oder elastische Festlegung, durch schließen eine Kammertüre (39) und/oder durch eine seitliche Klemmung herstellt.

3. Reinigungsvorrichtung nach Anspruch 1 und 2, **dadurch gekennzeichnet, dass** die Rahmung (36) ein Vorbau (55) oder eine Dichtung (51) ist oder die Vertiefung (34) eine Mulde oder eine Nute (57) ist und zumindest für die Hohlraumproduktereinigung mehrere Austrittsöffnungen (6) in Ausgestaltung von Öffnungen, Durchbrüchen, Adaptern, Ventilen (41), Sprühdüsen, selbstverschließbare Kappen und/oder Medienanschlüsse vorhanden sind.

4. Reinigungsvorrichtung nach einem der vorangegangenen Ansprüche, **dadurch gekennzeichnet, dass** zumindest eine Wandung oder der Boden eine doppelwandige Gerätekammer (27), ein doppelwandiger Maschineneinsatz oder ein Gitterkorb (1) ist, um Medium flächig zu verteilen oder auszuleiten und/oder in die Doppelwandung Heizstäbe oder die Doppelwand Sprühdüsen und/oder Austrittsöffnungen zur Produktreinigung besitzt.

5. Reinigungsvorrichtung nach einem der vorangegangenen Ansprüche, **dadurch gekennzeichnet, dass** zumindest eine Maschinenkammerwand (46) oder ein Maschinenkorb (1, 4) einen Vorbau (55) oder eine Rahmung (36) besitzt oder hiervon unabhängig ist, um einen Siebkorb (35) aufzunehmen und damit eine äußere flüssigkeitsbasierte Reinigung und/oder um eine Ultraschallreinigung in unterschiedlichen Etagen oder Höhen in einer Reinigungsmaschine durchzuführen.

6. Reinigungsvorrichtung nach einem der vorangegangenen Ansprüche, **dadurch gekennzeichnet, dass** diese zumindest ein Siebkorb ist und einen lösbaren Vorbau (55) besitzt oder ein Gitter (2), ein Gitterkorb (1, 4) oder ein Siebkorb (10, 35, 80, 110) in einem Reinigungsgerät und/oder außerhalb eines Reinigungsgerätes zumindest für die Produktreinigung einsetzbar ist.

7. Reinigungsvorrichtung nach einem der vorangegangenen Ansprüche, **dadurch gekennzeichnet, dass** die Vorrichtung einen Transponder oder einen Ultraschallabstandssensor enthält, damit die Kopplung und/oder die Aufbereitung dokumentierbar ist und/oder eine unkorrekte Kopplung angezeigt wird und/oder über diese Art der Kennung die Maschine steuerbar ist.

8. Reinigungsvorrichtung nach einem der vorangegangenen Ansprüche, **dadurch gekennzeichnet, dass** ein horizontal ausziehbarer Maschinenwagen mit Rollen, ein Korbgitter oder ähnliches mit Rollen oder Schienen aufweist oder als Korb oder Rahmen der einhängbar ist oder mehrere Gitter (2) oder Körbe (10, 35, 60, 70, 80, 90, 100, 110) eine teilbare Lagerungsebene herstellen.

9. Reinigungsvorrichtung nach Anspruch 8, **dadurch gekennzeichnet, dass** die Gitter (2) oder Körbe (10, 35, 60, 70, 80, 90, 100, 110) Anschlussadapter (6) und Produkthalterungen und/oder Materialausformung (61) zum Festlegen besitzen oder eine Spülvorrichtung durch Anpressen an eine Kammerwand (46) oder eine Maschinenwagenwand und/oder mit einem Vorbau (55) herstellen.

10. Reinigungsvorrichtung nach Anspruch 8 und 9, **dadurch gekennzeichnet, dass** steckbare und/oder flexibel ankoppelbare Medienverteiler (5, 24) in einer Reinigungskammer (27) zumindest festlegbar oder mobil am Gitter (2) oder Siebkorb aufnimmt, um die Reinigungsleistung zu ändern und/oder Anschlussmöglichkeiten für Hohlraumprodukte herzstellen.

11. Reinigungsvorrichtung nach einem der vorangegangenen Ansprüche, **dadurch gekennzeichnet, dass** ein Maschinenwagen (1) zumindest eine obere Lagerungsebene nach oben hin durch Hochklappen, herausnehmen oder Herausschieben geöffnet wird, um große, lange oder hohe Waschgüter reinigen zu können, um damit eine flexible Ausgestaltung eines Maschinenwagens (1) oder für ein einhängbaren Rahmen herstellen.

12. Reinigungsvorrichtung, nach einem der vorangegangenen Ansprüche, **dadurch gekennzeichnet, dass** die Gitter (2) oder Siebkörbe (10, 35, 60, 70, 80, 90, 100, 110) in unterschiedlichen Höhen in einer Reinigungskammer (27) angeordnet und/oder entnehmbar sind und zum Produkttransport und/oder zur Produktlagerung in einer Schublade oder in einem Sterilcontainer eingebracht oder mit einer Weichverpackung umhüllt werden.

13. Reinigungsvorrichtung nach einem der vorrangegangenen Ansprüchen, **dadurch gekennzeichnet, dass** hieran Spülleisten (5), Spülzylinder, Dreharme (24), Rohre (59), Sprühdüsen oder ähnliches zur Flüssigkeitsverteilung oder -weiterleitung angebracht sind, um damit Anschlussmöglichkeiten für die Hohlraumproduktreinigung herzustellen.

## Claims

1. Cleaning device for cleaning apparatus in which at least one machine insert, an insertion carriage, a grid, a grid basket or a screen basket or a combination of these is used, **characterized in that** the device first has a double walling (34, 36, 46, 52, 57) formed by a covering with a spaced chamber wall (46, 34, 36) or with a front structure (55) or frame (36, 51, 57) for distributing medium and/or ultrasonic energy via outlet openings (6, 41) and/or an ultrasonic transducer (42) for the purpose of cleaning products.

2. Cleaning device according to claim 1, **characterized in that** a framed (36, 51, 57) or a recessed surface (34) and/or just a single surface (52) with at least one existing outlet opening (6, 41) produces a chamber (44) by pressure via the weight force of the cleaning device, by mechanical or elastic fixing, by closing a chamber door (39) and/or by a lateral clamping.

3. Cleaning device according to claim 1 and 2, **characterized in that** the frame (36) is a front structure (55) or a seal (51) or the recess (34) is a trough or a groove (57) and in which several outlet openings (6), at least for cavity product cleaning, are provided in the form of openings, apertures, adapters, valves (41), spray nozzles, self-closable caps and/or media connections.

4. Cleaning device according to one of the above claims, **characterized in that** at least one wall or the bottom is a double-walled apparatus chamber (27), a double-walled machine insert or a grid basket (1) in order to distribute or discharge medium two-dimensionally and/or has heating rods in the double-wall or the double-wall has spray nozzles and/or outlet openings for product cleaning.

5. Cleaning device according to one of the preceding claims, **characterized in that** at least one machine chamber wall (46) or a machine basket (1, 4) has a front structure (55) or a frame (36) or is independent thereof, in order to receive a screen basket (35) and thus carry out an external liquid-based cleaning and/or an ultrasonic cleaning in different levels or heights in a cleaning machine.

6. Cleaning device according to one of the preceding claims, **characterized in that** this is at least one screen basket and has a detachable front structure (55) or a grid (2), a grid basket (1, 4) or a screen basket (10, 35, 80, 110) can be used in a cleaning apparatus and/or outside a cleaning apparatus at least for the product cleaning.

7. Cleaning device according to one of the preceding claims, **characterized in that** the device contains a transponder or an ultrasonic distance sensor so that the coupling and/or the processing can be documented and/or an incorrect coupling is indicated and/or the machine can be controlled via this type of identification.

8. Cleaning device according to one of the preceding claims, **characterized in that** a horizontally extendable machine carriage with rollers has a basket grille or the like with rollers or rails or can be suspended as a basket or frame or in which several grates (2) or baskets (10, 35, 60, 70, 80, 90, 100, 110) produce a divisible storage plane.

9. Cleaning device according to claim 8, **characterized in that** the grilles (2) or baskets (10, 35, 60, 70, 80, 90, 100, 110) have connection adapters (6) and product holders and/or material shaping (61) for fixing or produce a flushing device by pressing against a chamber wall (46) or a machine carriage wall and/or with a front structure (55).

10. Cleaning device according to claim 8 and 9, **characterized in that** pluggable and/or flexibly connectable media distributors (5, 24) can at least be fixed in a cleaning chamber (27) or received movably on the grille (2) or screen basket in order to change the cleaning performance and/or establish connection possibilities for hollow products.

11. Cleaning device according to one of the preceding claims, **characterized in that** a machine carriage (1) is opened at least one upper storage level upwards by folding up, taking out or pushing out in order to be able to clean large, long or high wash goods, so as to produce a flexible design of a machine carriage (1) or for a hookable frame.

12. Cleaning device, according to one of the above claims, **characterized in that** the grilles (2) or screen baskets (10, 35, 60, 60, 70, 80, 90, 100, 110) are arranged and/or removable at different heights in a cleaning chamber (27) and are introduced into a drawer or in a sterile container or wrapped in a soft packaging for product transport and/or product storage.

13. Cleaning device according to one of the preceding claims, **characterized in that** flushing bars (5), flushing cylinders, rotating arms (24), pipes (59), spray nozzles or the like are mounted thereon for distributing or passing on liquid in order to create connection possibilities for hollow product cleaning.

## Revendications

1. Dispositif de nettoyage pour appareils de nettoyage, dans lequel au moins un insert de machine, un chariot coulissant, une grille, une corbeille grillagée ou un tamis ou une combinaison de ceux-ci est utilisé, **caractérisé en ce que** le dispositif forme en premier une paroi double remplissable de fluide (34, 36, 46, 52, 57) par la couverture avec une paroi de chambre espacée (46, 34, 36) ou avec une saillie (55) ou un encadrement (36, 51, 57), pour distribuer à travers des ouvertures de sortie (6, 41) et/ou un émetteur d'ultrasons (42) du fluide et/ou les ultrasons pour le nettoyage de produits.

2. Dispositif de nettoyage selon la revendication 1, **caractérisé en ce qu'**une surface encadrée (36, 51, 57) ou une surface encastrée (34) et/ou une simple surface (52) avec au moins une ouverture de sortie (6, 41) existante forme une chambre (44) par pression au moyen de la force de poids du dispositif de nettoyage, par fixation mécanique ou élastique, par fermeture d'une porte de chambre (39) et/ou par un serrage latéral.

3. Dispositif de nettoyage selon la revendication 1 et 2, **caractérisé en ce que** l'encadrement (36) est une saillie (55) ou un joint d'étanchéité (51) ou que l'encastrement (34) est une cavité ou une rainure (57) et qu'au moins pour le nettoyage de produits creux plusieurs ouvertures de sortie (6) sont disponibles sous forme d'ouvertures, de perçages, d'adaptateurs, de soupapes (41), de buses de pulvérisation, de couvercles à fermeture automatique et/ou raccords de fluides.

4. Dispositif de nettoyage selon l'une quelconque des revendications précédentes, **caractérisé en ce qu'**au moins une paroi ou le fond est une chambre à outils (27), un insert de machine à double paroi ou une corbeille grillagée (1), pour distribuer uniformément sur une surface ou évacuer un fluide et/ou des éléments chauffants sont insérés dans la paroi double ou la paroi double est munie de buses de pulvérisation et/ou d'ouvertures de sortie pour le nettoyage de produits.

5. Dispositif de nettoyage selon l'une quelconque des revendications précédentes, **caractérisé en ce qu'**au moins une paroi de chambre de machine (46) ou une corbeille de machine (1, 4) possède une saillie (55) ou un encadrement (36) ou en est indépendant, pour recevoir un tamis (35) et effectuer ainsi un nettoyage extérieur à base de fluide et/ou pour effectuer un nettoyage aux ultrasons dans différents étages ou hauteurs dans une machine de nettoyage.

6. Dispositif de nettoyage selon l'une quelconque des revendications précédentes, **caractérisé en ce qu'**il est au moins un tamis et possède une saillie amovible (55) ou une grille (2), une corbeille grillagée (1, 4) ou un tamis (10, 35, 80, 110) est utilisable au moins pour le nettoyage de produits dans un appareil de nettoyage et/ou à l'extérieur d'un appareil de nettoyage.

7. Dispositif de nettoyage selon l'une quelconque des revendications précédentes, **caractérisé en ce que** le dispositif comprend un transpondeur ou un capteur de distance à ultrasons, afin de pouvoir documenter le couplage et/ou le traitement et/ou pour afficher un couplage incorrect et/ou pour permettre la commande de la machine à travers ce genre d'identification.

8. Dispositif de nettoyage selon l'une quelconque des revendications précédentes, **caractérisé en ce qu'**il comprend un chariot coulissant à roues extractible horizontalement, une corbeille grillagée ou similaire à roues ou rails ou **en ce qu'**il est une corbeille ou un cadre pouvant être accroché, ou **en ce que** plusieurs grilles (2) ou corbeilles (10, 35, 60, 70, 80, 90, 100, 110) forment un plan de stockage divisible.

9. Dispositif de nettoyage selon la revendication 8, **caractérisé en ce que** la grille (2) ou les corbeilles (10, 35, 60, 70, 80, 90, 100, 110) possèdent des adaptateurs de raccordement (6) et des supports de produits et/ou un relief de matière (61) pour la fixation, ou forment un dispositif de rinçage par pression sur une paroi de chambre (46) ou une paroi de chariot de machine et/ou avec une saillie (55).

10. Dispositif de nettoyage selon la revendication 8 et 9, **caractérisé en ce qu'**il reçoit de manière fixe ou mobile sur la grille (2) ou le tamis des distributeurs de fluide (5, 24) pouvant être accrochés et/ou couplés de manière flexible dans une chambre de nettoyage (27), afin de modifier la puissance de nettoyage et/ou pour former de possibilités de raccordement pour des produits creux.

11. Dispositif de nettoyage selon l'une quelconque des revendications précédentes, **caractérisé en ce qu'**au moins un plan de stockage supérieur d'un chariot de machine (1) peut est ouverte par relevage, retrait ou extraction afin de pouvoir nettoyer des produits grands, longs ou hauts, afin de créer ainsi une configuration flexible du chariot de machine (1) ou pour former un cadre pouvant être accroché.

12. Dispositif de nettoyage selon l'une quelconque des revendications précédentes, **caractérisé en ce que** la grille (2) ou les tamis (10, 35, 60, 70, 80, 90, 100, 110) peuvent être disposés à différentes hauteurs dans une chambre de nettoyage (27) et/ou en être retirés et qu'ils sont placés dans un tiroir ou un conteneur stérile ou enveloppés avec un emballage souple pour le transport et/ou le stockage de produits.

13. Dispositif de nettoyage selon l'une quelconque des revendications précédentes, **caractérisé en ce que** des réglettes de rinçage (5), des cylindres de rinçage, des bras rotatifs (24), des tubes (59), des buses de pulvérisation ou similaire pour la distribution ou le transport de fluides y sont installés, afin de créer des possibilités de raccordement pour le nettoyage de produits creux.
